# EUROPEAN PATENT SPECIFICATION

(11) **EP 3 735 587 B1**
(45) Date of publication and mention of the grant of the patent: **30.10.2024**
(21) Application number: 18885782.5
(22) Date of filing: 06.12.2018
(51) Int. Cl.: G01N 33/48, G01N 33/50, C12Q 1/6883

(54) **SYNCHRONIZED CELL CYCLE GENE EXPRESSION TEST FOR ALZHEIMER'S DISEASE AND RELATED THERAPEUTIC METHODS**
SYNCHRONISIERTER ZELLZYKLUS-GENEXPRESSIONSTEST FÜR MORBUS ALZHEIMER UND VERWANDTE THERAPEUTISCHE VERFAHREN
TEST D'EXPRESSION GÉNIQUE DE CYCLE CELLULAIRE SYNCHRONISÉ POUR LA MALADIE D'ALZHEIMER ET PROCÉDÉS THÉRAPEUTIQUES ASSOCIÉS

(30) Priority: 08.12.2017 US 201762596588 P
(43) Date of publication of application: 11.11.2020
(73) Proprietor: NeuroGX LLC, New York, NY 10017 (US)
(72) Inventor: CHIRILA, Florin Valentin, Morgantown West Virginia 26501 (US); ALKON, Daniel L., Chevy Chase Maryland 20815 (US)
(74) Representative: Abitz & Partner
(86) International application number: PCT/US2018/064322
(87) International publication number: WO 2019/113363

(56) References cited:
- WO-A1-2016/085943
- WO-A1-2016/144838
- WO-A1-2016/144838
- WO-A1-2016/170489
- WO-A1-2019/113277
- WO-A2-2005/003288
- US-A1- 2008 286 876
- US-A1- 2010 021 913
- US-A1- 2010 021 913
- US-A1- 2010 267 834
- US-A1- 2011 008 780
- US-A1- 2012 004 225
- US-A1- 2014 304 845
- US-A1- 2014 315 736
- US-A1- 2016 222 057
- Z NAGY: "The dysregulation of the cell cycle and the diagnosis of Alzheimer's disease", BIOCHIMICA ET BIOPHYSICA ACTA (BBA) - MOLECULAR BASIS OF DISEASE, vol. 1772, no. 4, 1 April 2007 (2007-04-01), pages 402 - 408, XP055049964, ISSN: 0925-4439, DOI: 10.1016/j.bbadis.2006.11.001
- KERBY SHEDDEN ET AL: "Analysis of cell-cycle-specific gene expression in human cells as determined by microarrays and double-thymidine block synchronization", 2 April 2002 (2002-04-02), pages 4379 - 4384, XP055093730, Retrieved from the Internet <URL:http://ukpmc.ac.uk/articlerender.cgi?artid=280370> [retrieved on 20131216], DOI: 10.1073/pnas.062569899
- HOOFT VAN HUIJSDUIJNEN R ED - MURGATROYD CHRISTOPHER: "ADAM 20 and 21; two novel human testis-specific membrane metalloproteases with similarity to fertilin-@a", GENE, ELSEVIER AMSTERDAM, NL, vol. 206, no. 2, 12 January 1998 (1998-01-12), pages 273 - 282, XP004108905, ISSN: 0378-1119, DOI: 10.1016/S0378-1119(97)00597-0
- YANG P ET AL: "The ADAMs family: Coordinators of nervous system development, plasticity and repair", PROGRESS IN NEUROBIOLOGY, ELSEVIER, AMSTERDAM, NL, vol. 79, no. 2, 1 June 2006 (2006-06-01), pages 73 - 94, XP027920049, ISSN: 0301-0082, [retrieved on 20060601]
- NOVAK ULRIKE: "ADAM proteins in the brain", JOURNAL OF CLINICAL NEUROSCIENCE, vol. 11, no. 3, 1 April 2004 (2004-04-01), GB, pages 227 - 235, XP055894328, ISSN: 0967-5868, DOI: 10.1016/j.jocn.2003.10.006
- MCKENZIE ANDREW T. ET AL: "Multiscale network modeling of oligodendrocytes reveals molecular components of myelin dysregulation in Alzheimer's disease", MOLECULAR NEURODEGENERATION, vol. 12, no. 1, 1 December 2017 (2017-12-01), XP055894333, Retrieved from the Internet <URL:https://www.ncbi.nlm.nih.gov/pmc/articles/PMC5674813/pdf/13024_2017_Article_219.pdf> DOI: 10.1186/s13024-017-0219-3
- PIETRZAK MACIEJ ET AL: "Gene expression profiling of brain samples from patients with Lewy body dementia", BIOCHEMICAL AND BIOPHYSICAL RESEARCH COMMUNICATIONS, vol. 479, no. 4, 1 October 2016 (2016-10-01), Amsterdam NL, pages 875 - 880, XP055894356, ISSN: 0006-291X, DOI: 10.1016/j.bbrc.2016.09.114
- SHPILKA TOMER ET AL: "Atg8: an autophagy-related ubiquitin-like protein family", GENOME BIOLOGY, vol. 12, no. 7, 1 January 2011 (2011-01-01), pages 226, XP055894345, ISSN: 1465-6906, DOI: 10.1186/gb-2011-12-7-226
- REDDY P HEMACHANDRA ET AL: "Differential loss of synaptic proteins in Alzheimer's disease: implications for synaptic dysfunction", JOURNAL OF ALZHEIMER`S DISEASE, IOS PRESS, NL, vol. 7, no. 2, 1 April 2005 (2005-04-01), pages 103 - 117, 173, XP009172020, ISSN: 1387-2877
- KONG WEI ET AL: "Independent component analysis of Alzheimer's DNA microarray gene expression data", MOLECULAR NEURODEGENERATION, BIOMED CENTRAL LTD, LO, vol. 4, no. 1, 28 January 2009 (2009-01-28), pages 5, XP021052336, ISSN: 1750-1326, DOI: 10.1186/1750-1326-4-5
- BIALOPIOTROWICZ ET AL.: "Cell cycle regulation distinguishes lymphocytes from sporadic and familial Alzheimers disease patients", NEUROBIOL AGING, vol. 32, no. 12, December 2011 (2011-12-01), pages 2319.e13 - 2319e.26, XP028311657
- HOSSINI ET AL.: "Induced pluripotent stem cell -derived neuronal cells from a sporadic Alzheimer's disease donor as a model for investigating AD-associated gene regulatory networks", BMC GENOMICS, vol. 16, no. 1, 14 February 2015 (2015-02-14), pages 84, XP021213627, DOI: 10.1186/s12864-015-1262-5

## Description

### Background of the Invention

Alzheimer's disease ("AD") has long been the subject of considerable efforts to develop accurate diagnostic methods, as well as therapeutic methods. Despite these efforts, there is an unmet need for methods of accurately diagnosing AD and differentiating it from non-Alzheimer's dementia ("non-ADD"). There is also an unmet need for effective methods of treating AD.

US2010/021913 A1 discloses a method for diagnosing Alzheimer's Disease using PKC-elicited gene expression profiles.

### Summary of the Invention

This invention provides a method for determining whether a human subject is afflicted with AD or non-ADD when the subject is suspected of being afflicted with AD or non-ADD, comprising the steps of
(a) synchronizing a population of suitable cells derived from the subject; and
(b) in the resulting synchronized cell population, measuring the expression level of a gene known to be differentially expressed between corresponding synchronized cells derived from AD patients and those derived from non-ADD patients, wherein the gene is PHBP8,
whereby (i) the subject is afflicted with AD if the expression level measured in step (b) is consistent with that gene's expression level in corresponding synchronized cells derived from AD patients, and (ii) the subject is afflicted with non-ADD if the expression level measured in step (b) is consistent with that gene's expression level in corresponding synchronized cells derived from non-ADD patients.

This invention also provides a method for determining whether a human subject is afflicted with AD or non-ADD when the subject is suspected of being afflicted with AD or non-ADD, comprising the steps of
(a) synchronizing a population of cultured skin cell fibroblasts derived from the subject, wherein the synchronizing comprises culturing the fibroblasts to over-confluence and then starving the resulting over-confluent fibroblasts; and
(b) in the resulting synchronized fibroblast population, measuring the expression level of each of genes AC004057.1, AC092651.1, ACP6, ADAM20, ASXL2, C2CD5, CARNS1, FAM149B1, GLIS3-AS1, IL18R1, LINC01393, LZIC, MAP1LC3B2, NHLH1, NORAD, NPPA-AS1_3, OSMR-AS1, PAN3, PHBP8, PSMB9, RAB3IP, RDH16, RFESDP1, RPL5, SCG2, SDHD, SHISA5, SLC45A3, SNHG14, TTC26, URB2, USMG5, WASF2, ZCWPW2, ZNF444, and ZNF70, wherein measuring the expression level of each gene comprises measuring the number of its RNA transcripts per number of total transcripts,
whereby (i) the subject is afflicted with AD if the expression levels measured in step (b) are consistent with the genes' expression levels in corresponding synchronized cells derived from AD patients, and (ii) the subject is afflicted with non-ADD if the expression levels measured in step (b) are consistent with the genes' expression levels in corresponding synchronized cells derived from non-ADD patients.

This invention further provides a method for determining whether a human subject is afflicted with AD or non-ADD when the subject is suspected of being afflicted with AD or non-ADD, comprising the steps of
(a) synchronizing a population of cultured immortalized B lymphocytes derived from the subject, wherein the synchronizing comprises culturing the lymphocytes to over-confluence and then starving the resulting over-confluent lymphocytes; and
(b) in the resulting synchronized lymphocyte population, measuring the expression level of each of genes AC004057.1, AC092651.1, ACP6, ADAM20, ASXL2, C2CD5, CARNS1, FAM149B1, GLIS3-AS1, IL18R1, LINC01393, LZIC, MAP1LC3B2, NHLH1, NORAD, NPPA-AS1_3, OSMR-AS1, PAN3, PHBP8, PSMB9, RAB3IP, RDH16, RFESDP1, RPL5, SCG2, SDHD, SHISA5, SLC45A3, SNHG14, TTC26, URB2, USMG5, WASF2, ZCWPW2, ZNF444, and ZNF70, wherein measuring the expression level of each gene comprises measuring the number of its RNA transcripts per number of total transcripts,
whereby (i) the subject is afflicted with AD if the expression levels measured in step (b) are consistent with the genes' expression levels in corresponding synchronized cells derived from AD patients, and (ii) the subject is afflicted with non-ADD if the expression levels measured in step (b) are consistent with the genes' expression levels in corresponding synchronized cells derived from non-ADD patients.

### Brief Description of the Figures

Figure 1
   This Figure, based on a first study ("Study 1"), shows statistically significant genes when comparing the AD group with the Non-ADD group. Study 1 revealed that there are 2103 statistically significant genes for a P level less than 0.1; 1099 statistically significant genes for a P level less than 0.05; 285 statistically significant genes for a P level less than 0.01; and 6 statistically significant genes for a P level less or equal than 0.001.
Figure 2
   This Figure, based on Study 1, shows the top 6 statistically significant genes (P<=0.001) for the 6 AD and 2 Non-ADD cases. Squares represent the AD population while circles represent the Non-ADD population.
Figure 3
   This Figure, based on Study 1, shows an example of the top 10 statistically significant genes (P<=0.01). (A) Raw TPM data showing with squares the AD population and with circles the Non-ADD population. (B) Average TPM data showing with squares the AD population and with circles the Non-ADD population. Error bars are standard deviations. (C) Percent change (%Ch) in gene expression when comparing the AD with control (Non-ADD), i.e., 100*(AD-Non-ADD)/Non-ADD.
Figure 4
   This Figure, based on Study 1, shows genes ranked 11 to 20 at the statistical significance of 1% overlap probability (P<=0.01). (A) Raw TPM data showing with squares the AD population and with circles the Non-ADD population. (B) Average TPM data showing with squares the AD population and with circles the Non-ADD population. Error bars are standard deviations. (C) Percent change (%Ch) in gene expression when comparing the AD with control (Non-ADD), i.e., 100*(AD-Non-ADD)/Non-ADD.
Figure 5
   This Figure, based on Study 1, shows genes ranked 21 to 30 at the statistical significance of 1% overlap probability (P<=0.01). (A) Raw TPM data showing with squares the AD population and with circles the Non-ADD population. (B) Average TPM data showing with squares the AD population and with circles the Non-ADD population. Error bars are standard deviations. (C) Percent change (%Ch) in gene expression when comparing the AD with control (Non-ADD), i.e., 100*(AD-Non-ADD)/Non-ADD.
Figure 6
   This Figure, based on Study 1, shows genes ranked 31 to 40 at the statistical significance of 1% overlap probability (P<=0.01). (A) Raw TPM data showing with squares the AD population and with circles the Non-ADD population. (B) Average TPM data showing with squares the AD population and with circles the Non-ADD population. Error bars are standard deviations. (C) Percent change (%Ch) in gene expression when comparing the AD with control (Non-ADD), i.e., 100*(AD-Non-ADD)/Non-ADD.
Figure 7
   This Figure, based on Study 1, shows the percent change (%Ch) in gene expression for the top 40 genes.
Figure 8
   This Figure, based on a second study ("Study 2"), shows the number of statistically significant differentially expressed genes for the training set (first cylinders-lighter shading) versus the number of statistically significant differentially expressed genes for the validation set (second cylinders-darker shading) for different levels of statistical significance P<0.001, 0.01, 0.05, and 0.10.
Figure 9
   This Figure, based on Study 2, shows gene networks for (A) PAN3, (B) PSMB9, (C) TTC26, (D) ZNF444, (E) NHLH1, (F) URB2 and (G) ADAM20.
Figure 10
   This Figure, based on Study 2, shows network measures for cross-validated genes: (A) number of edges; (B) average node degree; and (C) average local clustering coefficient.
Figure 11
   This Figure, based on Study 2, compares Non-ADD (n=3) with Non-Demented Controls (NDC; n=5), and shows the number of differentially expressed genes in the Non-ADD population when compared with the NDC population.

### Detailed Description of the Invention

### Definitions

In this application, certain terms are used which shall have the meanings set forth as follows.

As used herein, "administer", with respect to an agent, means to deliver the agent to a subject's body via any known method. Specific modes of administration include, without limitation, intravenous, oral, sublingual, transdermal, subcutaneous, intraperitoneal and intrathecal administration.

In addition, the various agents can be formulated using one or more routinely used pharmaceutically acceptable carriers. Such carriers are well known to those skilled in the art. For example, oral delivery systems include tablets and capsules. These can contain excipients such as binders (e.g., hydroxypropylmethylcellulose, polyvinyl pyrilodone, other cellulosic materials and starch), diluents (e.g., lactose and other sugars, starch, dicalcium phosphate and cellulosic materials), disintegrating agents (e.g., starch polymers and cellulosic materials) and lubricating agents (e.g., stearates and talc). Injectable drug delivery systems include, for example, solutions, suspensions, gels, microspheres and polymeric injectables, and can comprise excipients such as solubility-altering agents (e.g., ethanol, propylene glycol and sucrose) and polymers (e.g., polycaprylactones and PLGA's). Implantable systems include rods and discs and can contain excipients such as PLGA and polycaprylactone.

As used herein, "Alzheimer's disease" means a concurrent affliction with the following three symptoms: (i) dementia; (ii) amyloid plaques; and (iii) neurofibrillary tangles. Dementia can be diagnosed during life. Cerebral amyloid plaques and neurofibrillary tangles can, for example, be diagnosed during autopsy. This definition of Alzheimer's disease is the one provided by the National Institute of Neurological Disorders and Stroke (NINDS) of the National Institutes of Health (NIH), and is known as the "gold standard." All subjects from whom samples were taken and studied, and for which data are presented herein, are autopsy-confirmed AD and non-ADD patients.

As used herein, a gene's expression level is "consistent" with that gene's expression level in corresponding synchronized cells derived from AD patients if it is the same as, or close to, that expression level. For example, assume that gene X's TPM measure in synchronized cells derived from AD patients is 10 and its TPM measure is 100 in the same type of cells derived from non-ADD patients that are synchronized in the same way. A subject's gene X expression level would be consistent with gene X's AD expression level if it were, for example, below 50, below 40, below 30, below 20 or, ideally, 10 or lower.

As used herein, "culturing" lymphocytes is achieved, for example, by conducting the culturing at a temperature and in a growth factor milieu permissive of cell growth. In another embodiment, "culturing" lymphocytes is performed under conditions (e.g., those described herein for proliferation) that preserve lymphocyte viability. In one embodiment, the temperature, salinity and protein milieu permissive of cell growth is 37 °C, RPMI 1640 Medium with 10% fetal bovine serum ("FBS") and 1% penicillin ("PS"). In one embodiment of this invention, the lymphocyte-culturing step is performed for more than three hours. Preferably, the lymphocyte-culturing step is performed for more than six hours (e.g., overnight). B-lymphocyte can be cultured to over-confluence, i.e., high density/µl. The high density is determined as the plateau that is typically more then 90% in the growth curve. Then, the lymphocytes are starved overnight.

Methods for obtaining lymphocytes from a subject's blood are known, and include, for example, flow cytometry, Ficoll (a hydrophilic polysaccharide that separates layers of blood), and gradient centrifugation. Additionally, in the subject methods, the lymphocytes (e.g., B lymphocytes) can be used in immortalized or primary (i.e., non-immortalized) form. Methods for immortalizing lymphocytes (e.g., B lymphocytes) are known, and include, for example, treating the lymphocytes with Epstein-Barr virus ("EBV").

As used herein, "culturing" skin fibroblasts is achieved, for example, by conducting the culturing at a temperature and in a growth factor milieu permissive of cell growth. In another embodiment, "culturing" skin fibroblasts is performed under conditions (e.g., those described below for proliferation) that preserve skin fibroblasts viability. In one embodiment, the temperature, humidity and protein milieu permissive of cell growth is 37 °C, DMEM Medium with 10% fetal bovine serum ("FBS") and 1% penicillin ("PS"). In one embodiment of this invention, the skin fibroblast-culturing step is performed for more than three hours. Preferably, the skin fibroblast-culturing step is performed for more than six hours (e.g., overnight).

Methods for obtaining skin fibroblasts from a subject's blood are known, and include, for example, skin punch biopsy, and growing cells out of explants. When cell confluence reaches 100%, cells are passaged. Typically after two passages, fibroblasts are purified in a proportion greater than 95%.

As used herein, cells "derived" from a subject are cells that arise through culturing and/or other physical manipulation performed on cells directly removed from the subject. For example, cultured skin fibroblasts derived from a subject are those skin fibroblasts that arise through culturing a sample of skin cells (e.g., contained in a punch biopsy) directly removed from the subject.

As used herein, "diagnosing Alzheimer's disease", with respect to a symptomatic human subject, means determining that there is greater than 50% likelihood that the subject is afflicted with Alzheimer's disease. Preferably, "diagnosing Alzheimer's disease" means determining that there is greater than 60%, 70%, 80% or 90% likelihood that the subject is afflicted with Alzheimer's disease. As used herein, the phrase "determining whether the subject is afflicted with Alzheimer's disease" is synonymous with the phrase "diagnosing Alzheimer's disease."

As used herein, "diagnosing non-ADD", with respect to a symptomatic human subject, means determining that there is greater than 50% likelihood that the subject is afflicted with non-ADD. Preferably, "diagnosing non-ADD" means determining that there is greater than 60%, 70%, 80% or 90% likelihood that the subject is afflicted with non-ADD. As used herein, the phrase "determining whether the subject is afflicted with non-ADD" is synonymous with the phrase "diagnosing non-ADD."

As used herein, a gene is "differentially expressed between corresponding synchronized cells derived from AD patients and those derived from non-ADD patients" if, for example, the gene's TPM measure in synchronized cells derived from AD patients is different than in the same type of cells derived from non-ADD patients that are synchronized in the same way. For example, gene X would be differentially expressed between corresponding synchronized cells derived from AD patients and those derived from non-ADD patients if its TPM measure in synchronized cells derived from AD patients were 10 and its TPM measure were 100 in the same type of cells derived from non-ADD patients that are synchronized in the same way.

As used herein, an agent "favorably" affects the expression level of a gene whose expression level correlates with AD if it either decreases or increases that expression toward a level correlative with a non-AD state. For example, if the expression level of gene X is lower in an AD patient than in a non-afflicted patient, an agent favorably affecting the expression level of that gene would increase its expression level. Similarly, if the expression level of gene X is higher in an AD patient than in a non-afflicted patient, an agent favorably affecting the expression level of that gene would decrease its expression level.

As used herein, "measuring" the expression level of a gene means quantitatively determining the expression level via any means for doing so (e.g., Total RNA Sequencing, (20 million reads, 2x75bp PE)). Preferably, measuring the expression level of a gene is accomplished by measuring the number of RNA transcripts for that gene per million total RNA transcripts (i.e., "TPM" via FastQ data, and FPKM estimation per sample) present in the cell-derived RNA population being studied. For example, measuring the expression level of gene X in a synchronized cell population might yield a result of 50 TPM.

As used herein, a subject afflicted with "non-Alzheimer's dementia" means a subject showing dementia such as, for example, that which characterizes Parkinson's disease, Huntington's disease and frontotemporal dementia.

As used herein, a "population" of cells includes any number of cells permitting the manipulation and study required to assess gene expression. In one embodiment, the population of cells includes at least 1,000,000 cells. In another embodiment, the population of cells includes between 100,000 cells and 1,000,000 cells, between 10,000 cells and 100,000 cells, between 1,000 cells and 10,000 cells, between 100 cells and 1,000 cells, between 10 cells and 100 cells, and fewer than 10 cells (e.g., one cell).

As used herein, the term "subject" includes, without limitation, a mammal such as a human, a non-human primate, a dog, a cat, a horse, a sheep, a goat, a cow, a rabbit, a pig, a rat and a mouse. Where the subject is human, the subject can be of any age. For example, the subject can be 50 years or older, 55 years or older, 60 years or older, 65 or older, 70 or older, 75 or older, 80 or older, 85 or older, or 90 or older. The instant methods are envisioned for all subjects, preferably humans (and preferably symptomatic).

As used herein, a human subject who is "suspected of being afflicted with AD or non-ADD" is a subject displaying at least one symptom consistent with both AD and non-ADD, e.g., dementia.

As used herein, "synchronizing" a population of cells means placing at least a majority of cells in that population in the same cell cycle stage (namely, in the G1, S, G2 or M stage, and preferably in the G1, S or G2 stage). In one embodiment, synchronizing a population of cells means placing at least 60%, at least 70%, at least 80%, at least 90%, at least 95% or preferably at least 99% of cells in that population in the same cell cycle stage. In another embodiment, synchronizing a population of cells means placing the cells in that population in the same cell cycle stage that they would be in if cultured to over-confluence and then starved. Cell confluence followed by serum starvation typically arrests the cells in the G0/G1 stage [1-3].

Doses, i.e., "therapeutically effective amounts", include, for example, a single administration, and two or more administrations (i.e., fractions). In one embodiment, the therapeutically effective amount of a drug approved for a non-Alzheimer's indication is the dose and dosing regimen approved for that non-Alzheimer's indication.

As used herein, "treating" a subject afflicted with a disorder shall include, without limitation, (i) slowing, stopping or reversing the disorder's progression, (ii) slowing, stopping or reversing the progression of the disorder's symptoms, (iii) reducing the likelihood of the disorder's recurrence, and/or (iv) reducing the likelihood that the disorder's symptoms will recur. In the preferred embodiment, treating a subject afflicted with a disorder means (i) reversing the disorder's progression, ideally to the point of eliminating the disorder, and/or (ii) reversing the progression of the disorder's symptoms, ideally to the point of eliminating the symptoms.

The treatment of AD can be measured according to a number of clinical endpoints. These include, without limitation, (a) lowering, stabilizing or slowing progression of (i) dementia, (ii) synaptic loss, (iii) amyloid plaques and/or (iv) neurofibrillary tangles, and/or (b) favorably affecting the expression level of a gene whose expression level correlates with AD.

### Embodiments of the Invention

This invention provides accurate gene-based methods for determining whether a human subject is afflicted with AD or non-ADD when the subject is suspected of being afflicted with AD or non-ADD. The subject methods are based, at least in part, on the surprising discovery that synchronizing a patient's suitable cell population and then measuring the expression levels of genes that are differentially expressed between AD and non-ADD cells permits accurately diagnosing the patient as having either AD or non-ADD. Certain gene expression-altering agents for the use in treating AD are also described.

Specifically, this invention provides a method for determining whether a human subject is afflicted with AD or non-ADD when the subject is suspected of being afflicted with AD or non-ADD, comprising the steps of
(a) synchronizing a population of suitable cells derived from the subject; and
(b) in the resulting synchronized cell population, measuring the expression level of a gene known to be differentially expressed between corresponding synchronized cells derived from AD patients and those derived from non-ADD patients, wherein the gene is PHBP8,
whereby (i) the subject is afflicted with AD if the expression level measured in step (b) is consistent with that gene's expression level in corresponding synchronized cells derived from AD patients, and (ii) the subject is afflicted with non-ADD if the expression level measured in step (b) is consistent with that gene's expression level in corresponding synchronized cells derived from non-ADD patients.

In one embodiment of the subject method, the suitable cells derived from the subject are cultured skin cell fibroblasts. In another embodiment, the suitable cells derived from the subject are cultured B lymphocytes (preferably immortalized B lymphocytes).

Methods for synchronizing cell populations are known in the art. In one embodiment of the subject method, synchronizing the population of suitable cells comprises culturing the cells to over-confluence and then starving the resulting over-confluent cells.

Ideally in the subject method, the gene is known to be differentially expressed by a significant margin. In one embodiment, the gene is known to be differentially expressed by at least 50% between corresponding synchronized cells derived from AD patients and those derived from non-ADD patients. Preferably, the gene is known to be differentially expressed by at least 100% between corresponding synchronized cells derived from AD patients and those derived from non-ADD patients. Another way of expressing the degree of differential expression is "% change" or "%Ch", which is equal to [ADₑₓₚᵣₑₛₛᵢₒₙ - Non-ADDₑₓₚᵣₑₛₛᵢₒₙ / Non-ADDₑₓₚᵣₑₛₛᵢₒₙ].

In one embodiment, the gene expression levels set forth in Table 9, including PHBP8 and, one, two or more, three or more, four or more, are indicative of AD. In a preferred embodiment, the gene expression levels set forth in Table 10, including PHBP8 and, one, two or more, three or more, four or more, are indicative of AD. For example, as shown in Table 10, a PSMB9 expression level greater than 18 TPM is indicative of AD. In yet another embodiment, AD-indicative expression levels for each other gene disclosed herein are readily determined based on the data presented.

In a further preferred embodiment of the subject method, step (b) comprises measuring the expression levels of a plurality of genes including PHBP8, each gene being known to be differentially expressed between corresponding synchronized cells derived from AD patients and those derived from non-ADD patients. The plurality of genes can be of any suitable size, such as at least two genes, at least five genes, at least 20 genes, at least 100 genes, and at least 1,000 genes including PHBP8. Preferably, each gene of the plurality of genes is known to be differentially expressed by at least 50% (and more preferably by at least 100%) between corresponding synchronized cells derived from AD patients and those derived from non-ADD patients. In yet another preferred embodiment of the subject method, the plurality of genes comprises PHBP8 and one or more genes selected from the group consisting of AC004057.1, AC092651.1, ACP6, ADAM20, ASXL2, C2CD5, CARNS1, FAM149B1, GLIS3-AS1, IL18R1, LINC01393, LZIC, MAP1LC3B2, NHLH1, NORAD, NPPA-AS1_3, OSMR-AS1, PAN3, PSMB9, RAB3IP, RDH16, RFESDP1, RPL5, SCG2, SDHD, SHISA5, SLC45A3, SNHG14, TTC26, URB2, USMG5, WASF2, ZCWPW2, ZNF444, and ZNF70.

in the subject method where the expression levels of a plurality of genes are measured, the expression levels measured in step (b) are "consistent" with those in corresponding synchronized cells derived from AD patients if, for example, for at least a majority of gene expression levels measured, each such level is independently consistent with that gene's expression level in corresponding synchronized cells derived from AD patients.

In the subject method, measuring the expression level of a gene can be accomplished by any suitable method known in the art. In the preferred embodiment, measuring the expression level of a gene comprises measuring the number of that gene's RNA transcripts per number of total transcripts.

In a preferred embodiment, the subject invention provides a method for determining whether a human subject is afflicted with AD or non-ADD when the subject is suspected of being afflicted with AD or non-ADD, comprising the steps of
(a) synchronizing a population of cultured skin cell fibroblasts derived from the subject, wherein the synchronizing comprises culturing the fibroblasts to over-confluence and then starving the resulting over-confluent fibroblasts; and
(b) in the resulting synchronized fibroblast population, measuring the expression level of each of genes AC004057.1, AC092651.1, ACP6, ADAM20, ASXL2, C2CD5, CARNS1, FAM149B1, GLIS3-AS1, IL18R1, LINC01393, LZIC, MAP1LC3B2, NHLH1, NORAD, NPPA-AS1_3, OSMR-AS1, PAN3, PHBP8, PSMB9, RAB3IP, RDH16, RFESDP1, RPL5, SCG2, SDHD, SHISA5, SLC45A3, SNHG14, TTC26, URB2, USMG5, WASF2, ZCWPW2, ZNF444, and ZNF70, wherein measuring the expression level of each gene comprises measuring the number of its RNA transcripts per number of total transcripts,
whereby (i) the subject is afflicted with AD if the expression levels measured in step (b) are consistent with the genes' expression levels in corresponding synchronized cells derived from AD patients, and (ii) the subject is afflicted with non-ADD if the expression levels measured in step (b) are consistent with the genes' expression levels in corresponding synchronized cells derived from non-ADD patients.

In another preferred embodiment, the subject invention provides a method for determining whether a human subject is afflicted with AD or non-ADD when the subject is suspected of being afflicted with AD or non-ADD, comprising the steps of
(a) synchronizing a population of cultured immortalized B lymphocytes derived from the subject, wherein the synchronizing comprises culturing the lymphocytes to over-confluence and then starving the resulting over-confluent lymphocytes; and
(b) in the resulting synchronized lymphocyte population, measuring the expression level of each of genes AC004057.1, AC092651.1, ACP6, ADAM20, ASXL2, C2CD5, CARNS1, FAM149B1, GLIS3-AS1, IL18R1, LINC01393, LZIC, MAP1LC3B2, NHLH1, NORAD, NPPA-AS1_3, OSMR-AS1, PAN3, PHBP8, PSMB9, RAB3IP, RDH16, RFESDP1, RPL5, SCG2, SDHD, SHISA5, SLC45A3, SNHG14, TTC26, URB2, USMG5, WASF2, ZCWPW2, ZNF444, and ZNF70 wherein measuring the expression level of each gene comprises measuring the number of its RNA transcripts per number of total transcripts,
whereby (i) the subject is afflicted with AD if the expression levels measured in step (b) are consistent with the genes' expression levels in corresponding synchronized cells derived from AD patients, and (ii) the subject is afflicted with non-ADD if the expression levels measured in step (b) are consistent with the genes' expression levels in corresponding synchronized cells derived from non-ADD patients.

A method for determining whether a human subject is afflicted with AD, non-ADD or a disorder which is neither ("NDS") when the subject is suspected of being afflicted with AD or non-ADD is described, comprising the steps of
(a) synchronizing a population of suitable cells derived from the subject; and
(b) in the resulting synchronized cell population, measuring the expression level of a gene known to be differentially expressed between corresponding synchronized cells derived from AD patients, those derived from non-ADD patients and those derived from NDS subjects,
whereby (i) the subject is afflicted with AD if the expression level measured in step (b) is consistent with that gene's expression level in corresponding synchronized cells derived from AD patients, (ii) the subject is afflicted with non-ADD if the expression level measured in step (b) is consistent with that gene's expression level in corresponding synchronized cells derived from non-ADD patients, and (iii) the subject is afflicted with neither AD not non-ADD if the expression level measured in step (b) is consistent with that gene's expression level in corresponding synchronized cells derived from NDS subjects. The various embodiments of the diagnostic methods above for determining whether a human subject is afflicted with AD or non-ADD apply, *mutatis mutandis*, to this method.

An agent known to favorably affect the expression level of one or more genes whose expression levels correlate with Alzheimer's disease for the use in treating a human subject afflicted with Alzheimer's disease by administering to the subject a therapeutically effective amount of the agent is described. Preferably, the genes are selected from the group consisting of AC004057.1, AC092651.1, ACP6, ADAM20, ASXL2, C2CD5, CARNS1, FAM149B1, GLIS3-AS1, IL18R1, LINC01393, LZIC, MAP1LC3B2, NHLH1, NORAD, NPPA-AS1_3, OSMR-AS1, PAN3, PHBP8, PSMB9, RAB3IP, RDH16, RFESDP1, RPL5, SCG2, SDHD, SHISA5, SLC45A3, SNHG14, TTC26, URB2, USMG5, WASF2, ZCWPW2, ZNF444, and ZNF70. In one embodiment, the genes are selected from the group consisting of IL18R1, PSMB9, TTC26, WASF2, ACP6, CARNS1, NPPA-AS1_3, SCG2 and SDHD. In another embodiment, the gene is IL18R1, PSMB9, TTC26, WASF2, ACP6, CARNS1, NPPA-AS1_3, SCG2 or SDHD.

An agent selected from the group consisting of carfilzomib (Kyprolis^{®}, Onyx Pharmaceuticals), bortezomib (Velcade^{®}, Takeda Oncology), bumetanide (Bumex^{®}, Hoffman-La Roche), furosemide (Lasix^{®}), torsemide (Demadex^{®}), flavin mononucleotide, phosphoric acid, riboflavin, gamma-aminobutyric acid, adenosine monophosphate, histidine, L-arginine, cisplatin, clozapine, cyclosporin A, dexamethasone, etanercept, ethanol, filgrastim, glucose, haloperidol, heparin, infliximab, leflunomide, nitric oxide, oxygen, polyethylene glycol, prednisolone, progesterone, tacrolimus, thalidomide, zinc, calcitriol, calcium, serine, acetylcholine, capsaicin, dopamine, histamine, lithium, norepinephrine, succinic acid, formic acid, tromethamine, citric acid, 10Z-hymenialdisine (Tocris), JIB 04 (Tocris), CRT 0066101 (Tocris), celastrol, dihydroeponemycin, noradrenaline bitartrate (Tocris), or any other drug listed in Table 7 for the use in treating a human subject afflicted with Alzheimer's disease by administering to the subject a therapeutically effective amount of the agent, is described. In a preferred embodiment, the agent is carfilzomib which, in one embodiment, is administered in the manner stated on the FDA-approved label for one of its approved indications (e.g., in the manner approved for treating multiple myeloma, wherein the formulation is injectable and is administered at a dose of 30 mg or 60 mg). In another preferred embodiment, the agent is bortezomib which, in one embodiment, is administered in the manner stated on the FDA-approved label for one of its approved indications (e.g., in the manner approved for treating multiple myeloma, wherein the formulation is injectable and is administered at a dose of 3.5 mg, or 1.3 mg/m²). In another preferred embodiment, the agent is bumetanide which, in one embodiment, is administered in the manner stated on the FDA-approved label for one of its approved indications (e.g., in the manner approved for treating edema, wherein the formulation is oral and is administered at a dose of 0.5 mg, 1 mg or 2 mg daily, every other day, or daily for 3-4 days followed by a 1-2-day rest period). In another preferred embodiment, the agent is furosemide which, in one embodiment, is administered in the manner stated on the FDA-approved label for one of its approved indications (e.g., in the manner approved for treating edema or hypertension, wherein the formulation is oral and is administered at a dose of 20 mg, 40 mg, 60 mg or 80 mg per day (e.g., 40 mg 2x daily)). In another preferred embodiment, the agent is torsemide which, in one embodiment, is administered in the manner stated on the FDA-approved label for one of its approved indications (e.g., in the manner approved for treating edema or hypertension, wherein the formulation is oral and is administered at a dose of 5 mg, 10 mg, 15 mg or 20 mg per day).

In another preferred embodiment, the agent is any of cisplatin, clozapine, cyclosporin A, dexamethasone, etanercept, filgrastim, haloperidol, heparin, infliximab, leflunomide, prednisolone, progesterone, tacrolimus, thalidomide or calcitriol which, in one embodiment, is administered in the manner stated on the FDA-approved label for one of its approved indications.

As for each of 10Z-hymenialdisine, JIB 04, CRT 0066101, celastrol, dihydroeponemycin, noradrenaline bitartrate, and other non-FDA-approved drugs, the preferred route of administration is oral, and the preferred dosage is from 0.1 mg/kg to 100 mg/kg, from 1 mg/kg to 5 mg/kg, from 5 mg/kg to 10 mg/kg, from 10 mg/kg to 15 mg/kg, or from 15 mg/kg to 20 mg/kg.

This invention will be better understood by reference to the examples which follow, but those skilled in the art will readily appreciate that the specific examples detailed are only illustrative of the invention as described more fully in the claims which follow thereafter.

### Examples

### Example 1 - Study 1

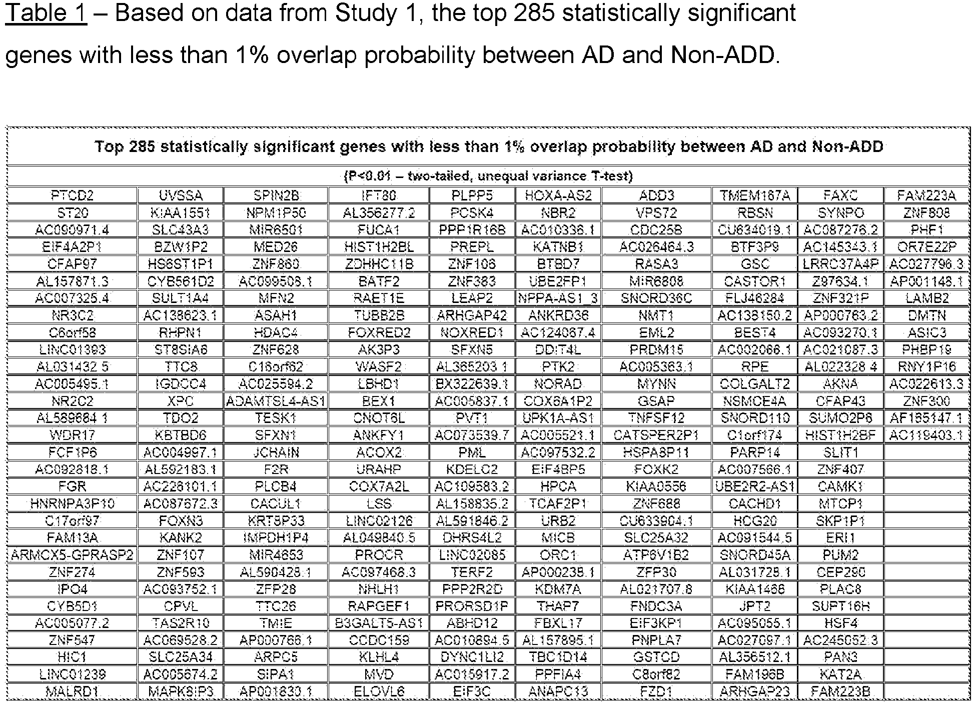

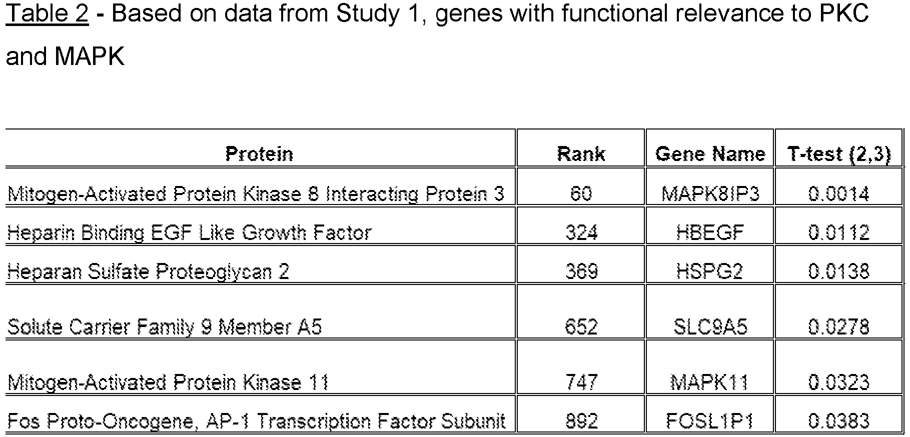

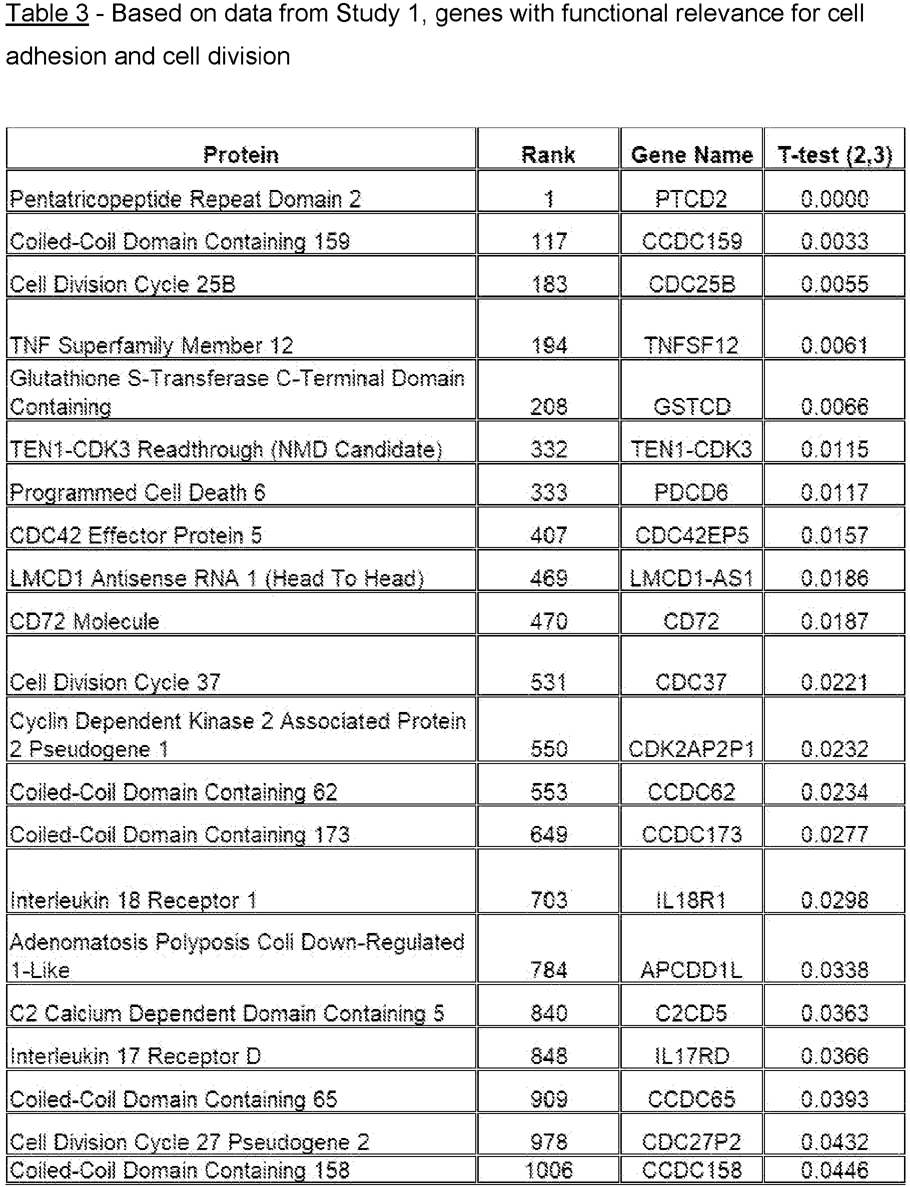

### Example 2 - Study 2; Synchronized Cell Cycle Gene Expression Test For Alzheimer's Disease: Cross-Validation of Genetic Differential Expression

The initial findings of the gene differential expression in synchronized skin fibroblasts, between the Alzheimer's Disease patients (AD; n=6) and the Non-Alzheimer's Disease Demented patients (Non-ADD; n=2), were cross-correlated with the second batch of samples (AD; n=2; Non-AD n=3). For the purpose of separating the two batches of samples, we called the first set of samples the "Training Set" and the second set of samples the "Validation Set."

### Methods

The genes were ranked in decreasing statistical significance order, i.e., with the highest statistical significance first (examples in Tables 4 and 5). The ranking is based on the t-test (two tailed, unequal variance) for the two groups of samples AD and Non-ADD. The comparison of the two lists of genes was made as described below.

### Results

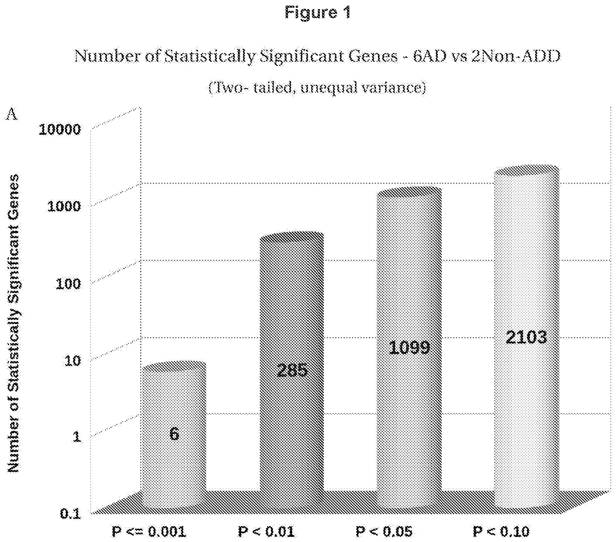

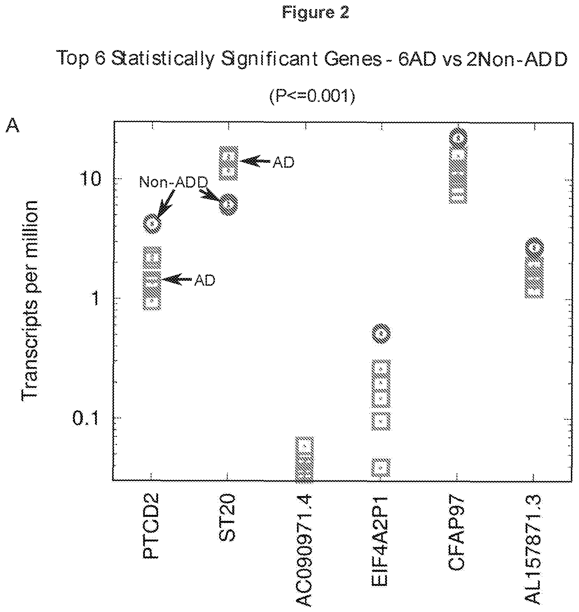

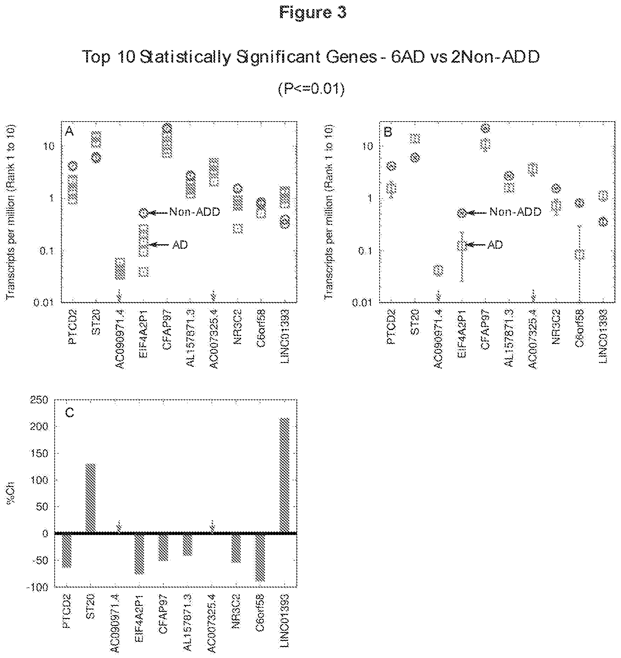

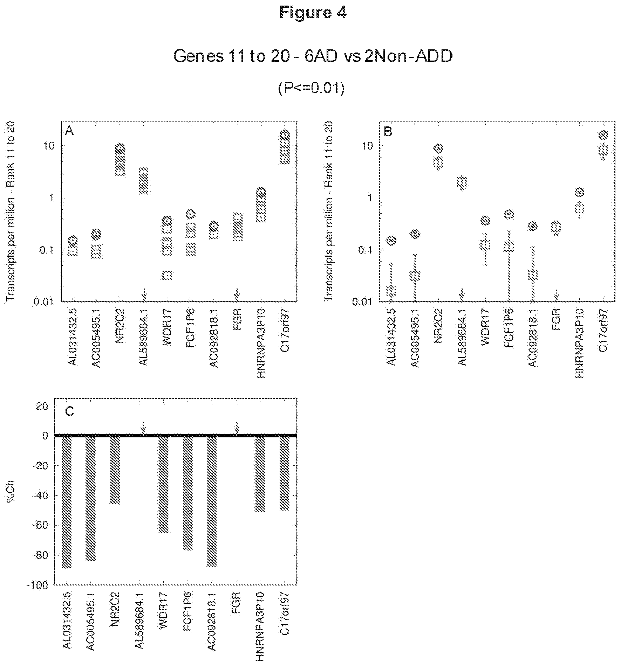

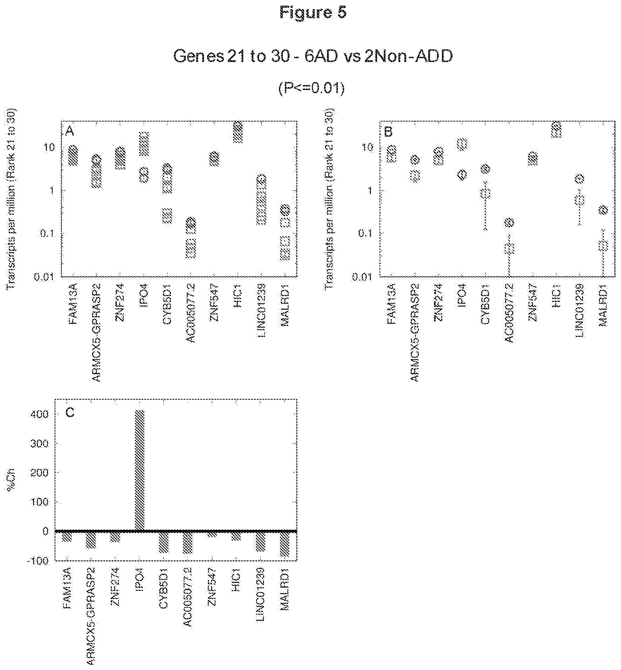

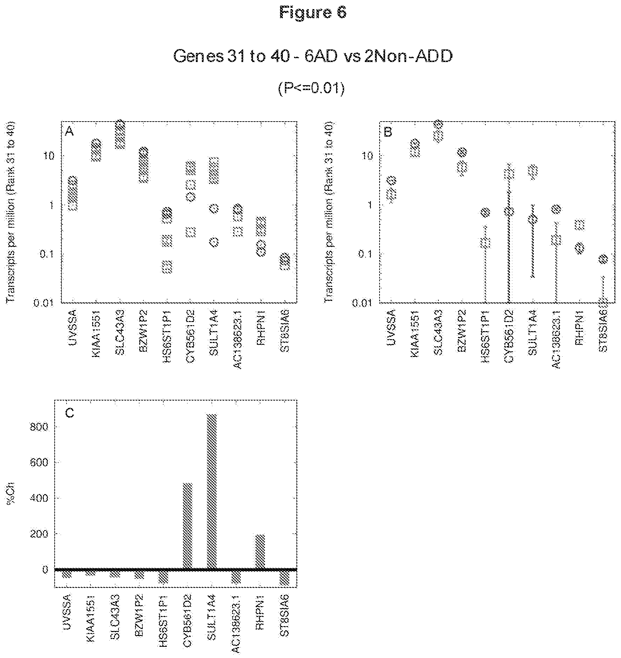

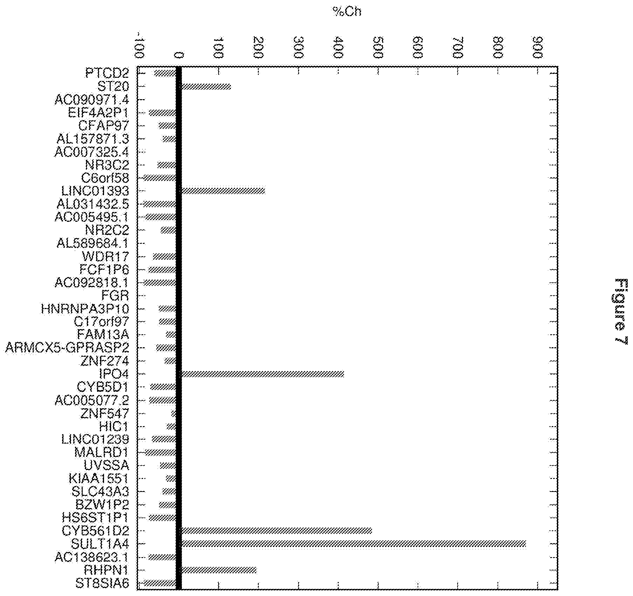

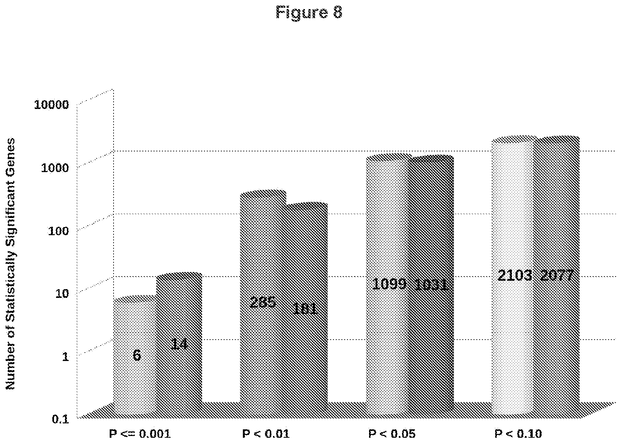

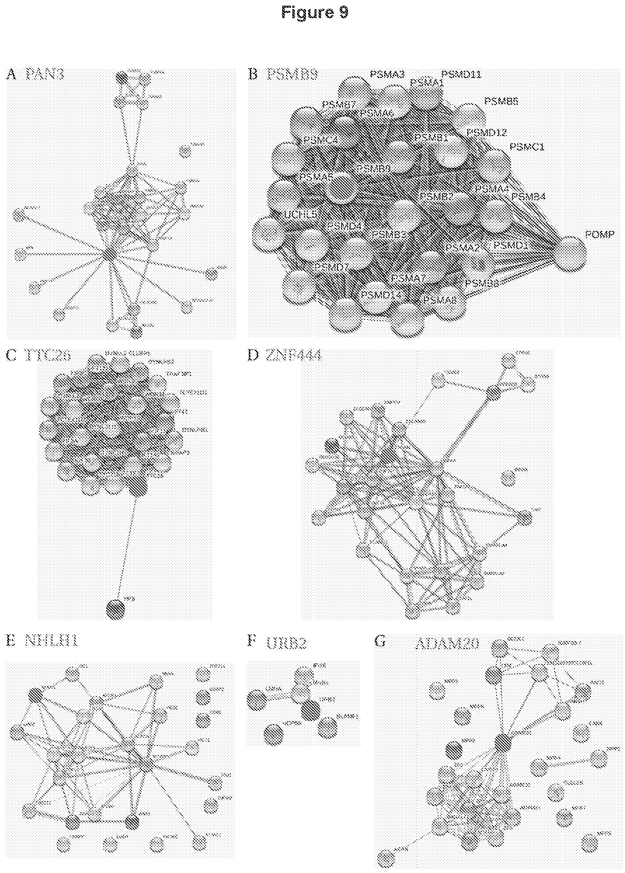

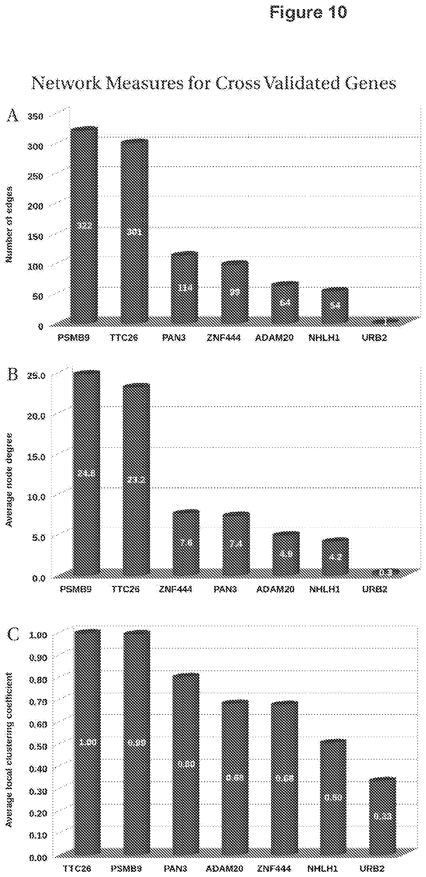

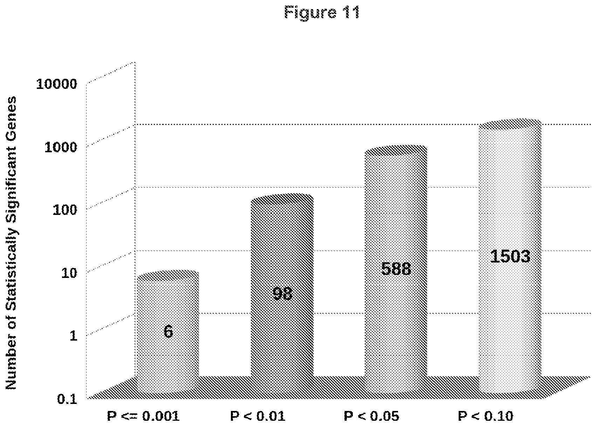

The number of statistically significant genes is similar in the training and validation sets (Fig. 8), with smaller differences for lower statistical significance (P<0.10) and larger differences for higher statistical significance (P<0.001). The larger difference for the higher statistical significance (P<0.001) could be due not only to the different number of samples in the validation set (5) when compared to the training set (8), but also to the different types of Non-ADD samples in the two sets. This difference suggests a high diversity of dysregulated pathways.

The majority of the genes (n=53) presented in Tables 4 and 5 are under highest statistical significance (P<0.001), and all of them are under high statistical significance (P<0.01). The presence of the first 40 genes from the training set (Table 4) was checked in the list of 2,077 genes from the validation set (P<0.10; Fig. 8). Similarly, the presence of the first 40 genes from the validation set (Table 5) was checked in the list of 2,103 genes from the training set (P<0.10; Fig. 8). The first 40 genes from Tables 4 and 5 are under highest statistical significance therefore it is very likely to have the highest impact in Alzheimer's disease detection, treatment, and pathways dysregulation. The cross-correlation of the first 40 genes in each set was made with a larger pool of genes from the opposite set (P<0.10) to accommodate the diversity in Non-ADD samples as well as to compensate for different numbers of samples in the validation (5) and training sets (8). However, in the end only the genes with similar statistical significance are considered as representing the core of dysregulation for AD.

The results of these initial findings in the highest statistically significant 40 genes suggests that about 81% of the genes which are dysregulated the training set are also dysregulated in the validation set. However, only about 7.5% of these genes show the same statistical significance in both training and validation set (Table 6).

Those genes showing the same statistical significance in the training and validation sets are at the core of the dysregulated pathways and will be very likely at the core of the genetic biomarkers for AD and at the core of the therapeutic targets for AD.

**Table 4 - Data for Differentially Expressed Genes from Study 2**

| First 40 differentially expressed genes in the Training Set (6AD, 2 Non-ADD). | | | | | |
|---|---|---|---|---|---|
| Training Set (6AD versus 2 Non-ADD) | | | | | |
| Rank | Gene name | T-test Two tailed Unequal Variance | Rank | Gene name | T-test Two tailed Unequal Variance |
| 1 | PTCD2 | 3.48E-05 | 21 | FAM13A | 5.15E-04 |
| 2 | ST20 | 6.09E-05 | 22 | ARMCX5-GPRASP2 | 5.17E-04 |
| 3 | AC090971.4 | 9.92E-05 | 23 | ZNF274 | 6.17E-04 |
| 4 | EIF4A2P1 | 1.28E-04 | 24 | IPO4 | 6.62E-04 |
| 5 | CFAP97 | 1.38E-04 | 25 | CYB5D1 | 6.96E-04 |
| 6 | AL157871.3 | 1.44E-04 | 26 | AC005077.2 | 7.29E-04 |
| 7 | AC007325.4 | 2.26E-04 | 27 | ZNF547 | 7.57E-04 |
| 8 | NR3C2 | 2.37E-04 | 28 | HIC1 | 7.58E-04 |
| 9 | C6orf58 | 2.38E-04 | 29 | LINC01239 | 7.59E-04 |
| 10 | LINC01393 | 2.58E-04 | 30 | MALRD1 | 7.87E-04 |
| 11 | AL031432.5 | 2.87E-04 | 31 | UVSSA | 8.09E-04 |
| 12 | AC005495.1 | 2.96E-04 | 32 | KIAA1551 | 8.13E-04 |
| 13 | NR2C2 | 3.07E-04 | 33 | SLC43A3 | 8.17E-04 |
| 14 | AL589684.1 | 3.26E-04 | 34 | BZW1 P2 | 8.21E-04 |
| 15 | WDR17 | 3.81E-04 | 35 | HS6ST1P1 | 8.89E-04 |
| 16 | FCF1P6 | 4.04E-04 | 36 | CYB561D2 | 9.04E-04 |
| 17 | AC092818.1 | 4.30E-04 | 37 | SULT1A4 | 9.57E-04 |
| 18 | FGR | 4.36E-04 | 38 | AC138623.1 | 9.58E-04 |
| 19 | HNRNPA3P10 | 4.41E-04 | 39 | RHPN1 | 9.68E-04 |
| 20 | C17orf97 | 4.73E-04 | 40 | ST8SIA6 | 9.82E-04 |

**Table 5 - Data for Differentially Expressed Genes from Study 2**

| First 40 differentially expressed genes in the Validation Set (2AD, 3 Non-ADD) | | | | | |
|---|---|---|---|---|---|
| Validation Set (2 AD versus 3 Non-ADD) | | | | | |
| Rank | Gene name | T-test Two tailed Unequal Variance | Rank | Gene name | T-test Two tailed Unequal Variance |
| 1 | RPL13AP6 | 2.36E-05 | 21 | bP-21264C1.2 | 1.31E-03 |
| 2 | ARHGEF7 | 7.59E-05 | 22 | FRMD5 | 1.37E-03 |
| 3 | ZNF623 | 9.37E-05 | 23 | ATOX1 | 1.39E-03 |
| 4 | MYL12B | 3.47E-04 | 24 | ZCWPW1 | 1.43E-03 |
| 5 | RP11-500C11.3 | 3.79E-04 | 25 | NENF | 1.46E-03 |
| 6 | EEF1A1P9 | 4.17E-04 | 26 | RPS15AP38 | 1.61E-03 |
| 7 | EIF3M | 5.39E-04 | 27 | RP11-568N6.1 | 1.69E-03 |
| 8 | NDUFB6 | 5.93E-04 | 28 | ZNF786 | 1.71E-03 |
| 9 | PGAM4 | 7.27E-04 | 29 | ZNF3 | 1.74E-03 |
| 10 | XXYLT1-AS2 | 8.29E-04 | 30 | AP000688.14 | 1.75E-03 |
| 11 | PIGX | 8.60E-04 | 31 | RP5-1125A11.6 | 1.84E-03 |
| 12 | FAM71F2 | 8.74E-04 | 32 | HAUS2 | 1.87E-03 |
| 13 | MPLKIP | 9.28E-04 | 33 | NDUFS1 | 1.95E-03 |
| 14 | NDUFA8 | 9.30E-04 | 34 | CAPNS1 | 2.05E-03 |
| 15 | TCP10L | 1.06E-03 | 35 | STEAP4 | 2.08E-03 |
| 16 | ATG9B | 1.09E-03 | 36 | PAN3 | 2.09E-03 |
| 17 | FAM229B | 1.15E-03 | 37 | RP5-940J5.6 | 2.10E-03 |
| 18 | RPS18P12 | 1.15E-03 | 38 | RP11-266K4.14 | 2.10E-03 |
| 19 | RP3-340B19.2 | 1.16E-03 | 39 | ATP5L | 2.21E-03 |
| 20 | SHFM1 | 1.27E-03 | 40 | PSMB9 | 2.21E-03 |

**Table 6 - Data for Differentially Expressed Genes from Study 2**

| Differentially expressed genes with similar statistical significance (P<0.05; n=36) in the Training and Validation sets. | | | |
|---|---|---|---|
| Cross-Validated Genes under Statistical Significance of P<0.05 | | | |
| Number | Gene name | T-test Training | T-test Validation |
| 1 | AC004057.1 | 0.0246 | 0.0199 |
| 2 | AC092651.1 | 0.0307 | 0.0332 |
| 3 | ACP6 | 0.0332 | 0.0169 |
| 4 | ADAM20 | 0.0321 | 0.0082 |
| 5 | ASXL2 | 0.0397 | 0.0298 |
| 6 | C2CD5 | 0.0363 | 0.0256 |
| 7 | CARNS1 | 0.0281 | 0.0316 |
| 8 | FAM149B1 | 0.0370 | 0.0150 |
| 9 | GLIS3-AS1 | 0.0206 | 0.0409 |
| 10 | IL18R1 | 0.0298 | 0.0399 |
| 11 | LINC01393 | 0.0003 | 0.0115 |
| 12 | LZIC | 0.0338 | 0.0479 |
| 13 | M AP1LC3B2 | 0.0260 | 0.0271 |
| 14 | NHLH1 | 0.0032 | 0.0119 |
| 15 | NORAD | 0.0050 | 0.0424 |
| 16 | NPPA-AS1_3 | 0.0048 | 0.0080 |
| 17 | OSMR-AS1 | 0.0393 | 0.0241 |
| 18 | PAN3 | 0.0088 | 0.0021 |
| 19 | PHBP8 | 0.0256 | 0.0198 |
| 20 | PSMB9 | 0.0378 | 0.0022 |
| 21 | RAB3IP | 0.0137 | 0.0186 |
| 22 | RDH16 | 0.0117 | 0.0434 |
| 23 | RFESDP1 | 0.0237 | 0.0043 |
| 24 | RPL5 | 0.0220 | 0.0422 |
| 25 | SCG2 | 0.0408 | 0.0295 |
| 26 | SDHD | 0.0328 | 0.0463 |
| 27 | SHISA5 | 0.0188 | 0.0169 |
| 28 | SLC45A3 | 0.0265 | 0.0359 |
| 29 | SNHG14 | 0.0292 | 0.0259 |
| 30 | TTC26 | 0.0023 | 0.0269 |
| 31 | URB2 | 0.0051 | 0.0219 |
| 32 | USMG5 | 0.0384 | 0.0340 |
| 33 | WASF2 | 0.0027 | 0.0476 |
| 34 | ZCWPW2 | 0.0145 | 0.0107 |
| 35 | ZNF444 | 0.0158 | 0.0056 |
| 36 | ZNF70 | 0.0301 | 0.0311 |

**Table 7 - Top Cross-Validated Genes (P<0.05); Drugs and Disorders (Study 2)**

| Top Cross-Validated Genes (P<0.05); Drugs and Known Disorders | | | | |
|---|---|---|---|---|
| # | Gene name | Drugs | Company | Known Disorders |
| 1 | AC004057. 1 alias for RPS26P25 | NA | NA | Increased risk of cardiovascular disease (CVD) |
| 2 | AC092651. 1 alias for LOC10042 0889 | NA | NA | NA |
| 3 | ACP6 | Flavin Mononucleotide (Approved, Investigational), Phosphoric acid (Approved), Riboflavin (Approved, Investigational), 4-Nitropheno (Experimental) | Pharma, Nutra | Schizophrenia, congenital heart disease (CHD) |
| 4 | ADAM20 | NA | NA | NA |
| 5 | ASXL2 | NA | NA | Shashi-Pena syndrome; therapy-related myelodysplastic syndrome; ASXL2 and ASXL1 genes were predicted cancer-associated genes |
| 6 | C2CD5 | NA | NA | Dynamically associated with GLUT4-containing glucose storage vesicles (GSV) and plasma membrane in response to insulin stimulation |
| 7 | CARNS1 | Gamma-Aminobutyric acid (Approved, Investigational), Phosphoric acid (Approved), Adenosine monophosphate (Approved, Investigational), Histidine (Approved), L-Arginin (Approved) | Pharma, Nutra | NA |
| 8 | FAM149B1 | NA | NA | NA |
| 9 | GLIS3-AS1 | NA | NA | Diabetes mellitus, Neonatal, with congenital hypothyroidism; ndh ndh syndrome neonatal diabetes mellitus with congenital hypothyroidism neonatal diabetes-congenital hypothyroidism-congenital glaucoma-hepatic fibrosis-polycystic kidneys syndrome |
| 10 | IL18R1 | (43) Drugs for IL18R1 Gene, Cisplatin (Approved), Clozapine (Approved), Cyclosporin A (Approved, Investigational), Dexamethasone (Approved, Investigational), Etanercept (Approved, Investigational), Ethanol (Approved), Filgrastim (Approved), glucose (Approved), Haloperidol (Approved), Heparin (Approved, Investigational), Infliximab (Approved), Leflunomide (Approved, Investigational), Nitric Oxide (Approved), Oxygen (Approved), Polyethylene glycol (Approved), Prednisolone (Approved), Progesterone (Approved), Tacrolimus (Approved, Investigational), Thalidomide (Approved, Investigational), Zinc (Approved, Investigational), Calcitriol (Approved), calcium (Approved), Serine (Approved), cyclic amp (Experimental), thymidine (Experimental, Investigational), Vesnarinone (Investigational), Ceramide, estrogen, LY294002, mometasone furoate, NMDA, Progestins, Rapamycin, alanine, arginine, cysteine, glutamine, leucine, phenylalanine, proline, threonine, tyrosine | Pharma, Nutra | Ordinary smallpox, Variola, growth hormone insensitivity syndrome, pituitary dwarfism, growth hormone receptor deficiency, laron dwarfism, laron-type isolated somatotropin defect, laron-type dwarfism, laron type pituitary dwarfism, primary growth hormone insensitivity, primary growth hormone resistance, gh-r deficiency, growth hormone receptor defect, laron-type pituitary dwarfism, laron-type short stature, primary gh resistance, severe gh insensitivity, complete growth hormone insensitivity, gh receptor deficiency, primary gh insensitivity, short stature due to growth hormone resistance, lars, acute basophilic leukemia, ehrlichiosis chafeensis, hme human ehrlichial infection, human monocytic type, pneumoconiosis, black lung, coal miner's pneumoconiosis, coal workers' lung, coal workers' pneumoconiosis, melanoedema, coal worker's pneumoconiosis, black lung disease, coal workers pneumoconiosis. |
| 11 | LINC01393 | NA | NA | NA |
| 12 | LZIC | NA | NA | Thiazolidinedione-induced edema in the Diabetes |
| 13 | MAP1LC3 B2 | NA | NA | NA |
| 14 | NHLH1 | NA | NA | cleft palate, isolated, physical disorder, orofacial cleft, cleft lip/palate-ectodermal dysplasia syndrome, split-hand/foot malformation |
| 15 | NORAD | Noradrenaline bitartrate | Tocris | pancreatic cancer, bladder cancer, esophageal cancer, breast cancer, colorectal cancer |
| 16 | NPPA-AS1_3 | Bumetanide (Approved), Furosemide (Approved), Torsemide (Approved) | Genentech, Inc., Validus Pharmaceuti cals LLC, Leo Pharma, Apotex Corporation, Sanis Health Inc, Watson Labs, Roche | atrial fibrillation, atrial standstill, atrial cardiomyopathy with heart block |
| 17 | OSMR-AS1 | NA | NA | NA |
| 18 | PAN3 | 10Z-Hymenialdisine, JIB 04, AZD 1208, G 5555, CRT 0066101 | Tocris | NA |
| 19 | PHBP8 | NA | NA | NA |
| 20 | PSMB9 | Carfilzomib (Approved, Investigational), Bortezomib (Approved, Investigational), Kyprolis (Approved July 2012), Celastrol, Dihydroeponemycin | Amgen, Teva, Pfizer | eosinophilic variant of chromophobe renal cell carcinoma, nasopharyngeal disease, waterhouse-friderichsen syndrome, cardiac sarcoidosis, epstein-barr virus-associated gastric carcinoma |
| 21 | RAB3IP | NA | NA | Involved in actin remodeling and polarized membrane transport. |
| 22 | RDH16 | Farnesol (Experimental), NAD, Androstanediol, Androsterone | NA | NA |
| 23 | RFESDP1 | NA | NA | NA |
| 24 | RPL5 | Zinc (Approved, Investigational), | NA | NA |
| 25 | SCG2 | Calcium (Approved), Acetylcholine (Approved), Capsaicin (Approved), Dexamethasone (Approved, Investigational), Dopamine (Approved), Glucose (Approved), Histamine (Approved, Investigational), Lithium (Approved), Norepinephrine (Approved), Cyclic amp (Experimental), ATP (Investigational), 5-Hydroxytryptamine, Forskolin, Cysteine, Tyrosine | Pharma, Nutra | Intracranial primitive neuroectodermal tumor (intracranial pnet;intracranial primitive neuroectodermal neoplasm), Lymph node cancer (lymph node neoplasm, neoplasm of lymph node), Collagenous colitis (microscopic colitis, collagenous type colitis, collagenous), Neuroendocrine tumor (neuroendocrine neoplasm, neuroendocrine carcinoma, neuroendocrine cancer, neuroendocrine neoplasia, carcinoma neuroendocrine, neuroendocrine tumors, carcinoma neuroendocrine), Pheochromocytoma (pheochromocytoma, susceptibility to pheochromocytoma, modifier of sporadic pheochromocytoma/secreti ng paraganglioma chromaffin cell tumor medullary chromaffinoma medullary paraganglioma pheochromoblastoma pcc chromaffin cell neoplasm pheochromocytoma, malignant) |
| 26 | SDHD | Succinic acid (Approved), Formic acid (Approved, Experimental, Investigational), Tromethamine (Approved), Citric Acid (Approved) | Pharma, Nutra | Paraganglioma and gastric stromal sarcoma, Paragangliomas, Cowden syndrome, Mitochondrial complex ii deficiency, Carcinoid tumors, intestinal |
| 27 | SHISA5 | NA | NA | Vasculopathy, retinal, with cerebral leukodystrophy,aicardi-goutieres syndrome 1 (cree encephalitis, aicardi-goutières syndrome, ags, encephalopathy with basal ganglia calcification, encephalopathy with intracranial calcification and chronic lymphocytosis of cerebrospinal fluid, encephalopathy, familial infantile, with calcification of basal ganglia and chronic cerebrospinal fluid lymphocytosis pseudotoxoplasmosis syndrome familial infantile encephalopathy with intracranial calcification and chronic cerebrospinal fluid lymphocytosis) |
| 28 | SLC45A3 | NA | NA | Prostate cancer, Suppression of tumorigenicity 12 (st12; prostate adenocarcinoma 1; pac1), male reproductive organ cancer. |
| 29 | SNHG14 | NA | NA | Angelman syndrome (happy puppet syndrome), Prader-willi syndrome (prader-labhart-willi syndrome), Gastric cancer |
| 30 | TTC26 | NA | NA | joubert syndrome (Joubert-boltshauser syndrome, Cerebelloparenchymal disorder, cerebellar vermis agenesis, agenesis of cerebellar vermis, cerebello-oculo-renal syndrome, familial aplasia of the vermis, cerebello-oculo-renal syndrome 1.) |
| 31 | URB2 | NA | NA | Hepatocellular carcinoma*(), Buruli ulcer (buruli ulcer, susceptibility to mycobacterium ulcerans, mycobacterium ulcerans, bairnsdale ulcer, daintree ulcer, mossman ulcer, searl ulcer) |
| 32 | USMG5 alias for ATP5MD | NA | NA | NA |
| 33 | WASF2 | Tyrosine | NA | wiskott-aldrich syndrome (eczema-thrombocytopenia-immunodeficiency syndrome), narcissistic personality disorder, substance abuse, tobacco addiction, avoidant personality disorder (anxious personality disorder) |
| 34 | ZCWPW2 | NA | NA | NA |
| 35 | ZNF444 | NA | NA | chondrosarcoma, extraskeletal myxoid (extraskeletal myxoid chondrosarcoma, myxoid extraosseous chondrosarcoma) |
| 36 | ZNF70 | NA | NA | NA |

**Table 8 - Protein Networks (Study 2)**

| Number | Gene name | Number of nodes | Number of edges | Average node degree | Average local clustering coefficient |
|---|---|---|---|---|---|
| 1 | AC004057.1 | NA | NA | NA | NA |
| 2 | AC092651.1 | NA | NA | NA | NA |
| 3 | ADAM20 | 26 | 64 | 4.92 | 0.681 |
| 4 | ASXL2 | 26 | 212 | 16.3 | 0.876 |
| 5 | C2CD5 | 26 | 239 | 18.4 | 0.98 |
| 6 | CARNS1 | 26 | 209 | 16.1 | 0.84 |
| 7 | FAM149B1 | 2 | 1 | 1 | 1 |
| 8 | GLIS3-AS1FAM149B1 | NA | NA | NA | NA |
| 9 | IL18R1 | 20 | 90 | 9 | 0.768 |
| 10 | LINC01393 | NA | NA | NA | NA |
| 11 | LINC01393 | NA | NA | NA | NA |
| 12 | LZIC | 2 | 1 | 1 | 1 |
| 13 | MAP1LC3B2 | NA | NA | NA | NA |
| 14 | NHLH1 | 26 | 54 | 4.15 | 0.504 |
| 15 | NORAD | NA | NA | NA | NA |
| 16 | NPPA-AS1_3 | NA | NA | NA | NA |
| 17 | OSMR-AS1 | NA | NA | NA | NA |
| 18 | PAN3 | 31 | 114 | 7.35 | 0.801 |
| 19 | PHBP8 | NA | NA | NA | NA |
| 20 | PSMB9 | 26 | 322 | 24.8 | 0.992 |
| 21 | RAB3IP | 26 | 301 | 23.2 | 0.997 |
| 22 | RDH16 | 26 | 39 | 3 | 0.613 |
| 23 | RFESDP1 | NA | NA | NA | NA |
| 24 | RPL5 | 26 | 323 | 24.8 | 0.994 |
| 25 | SCG2 | 18 | 27 | 3 | 0.586 |
| 26 | SDHD | 26 | 115 | 8.85 | 0.769 |
| 27 | SHISA5 | 10 | 5 | 1 | 0.6 |
| 28 | SLC45A3 | 14 | 6 | 0.857 | 0.381 |
| 29 | SNHG14 | NA | NA | NA | NA |
| 30 | TTC26 | 26 | 301 | 23.2 | 0.997 |
| 31 | URB2 | 6 | 1 | 0.333 | 0.333 |
| 32 | USMG5 | 26 | 325 | 25 | 1 |
| 33 | WASF2 | 51 | NA | NA | NA |
| 34 | ZCWPW2 | NA | NA | NA | NA |
| 35 | ZNF444 | 26 | 99 | 7.62 | 0.675 |
| 36 | ZNF70 | 26 | 126 | 9.69 | 0.612 |

### Example 3 - Study 2; TPM Values

### Reference Intervals

The average and standard deviations were calculated for the Transcripts Per Million (TPM) values for each of the two groups - Alzheimer's disease (AD) and Non-Alzheimer's Disease Demented (Non-ADD) for each gene. The reference intervals were then calculated according to Horn and Pesce (Reference Intervals: A User's Guide. Paul S. Horn and Amadeo J. Pesce. Washington, DC: AACC Press, 2005, ISBN 1-59425-035-9) as the average plus minus two standard deviations. The reference intervals calculated in this way assure that 95% of all the possible values in each population (AD or non-ADD) are considered.

### Gap between AD and Non-ADD

If there is no overlap between the reference intervals of AD and Non-ADD, there is a gap between the two bell-shaped curves and that indicates unequivocal diagnosis.

If there is an overlap in the reference intervals for AD and Non-ADD (light grey in Table 9), then there is a possibility of having a false positive or a false negative in the diagnosis. The genes that show overlap in the reference intervals, i.e., no gap (light grey) were eliminated from the final vector diagnosis. The genes that show an average of zero in one of the groups, either in the AD group or in the Non-ADD group, were also eliminated.

### Cut-off for each gene

The cut-offs for each of the remaining 26 genes (Table 10) was determined as the middle of the gap in the reference intervals.

### Genetic Vector AD Diagnosis

The AD diagnosis is based on the 26 components/genes of the vector. For each one of the components, the greater than (>) or smaller than (<) the cut-off value is indicated for each gene, in the last column.

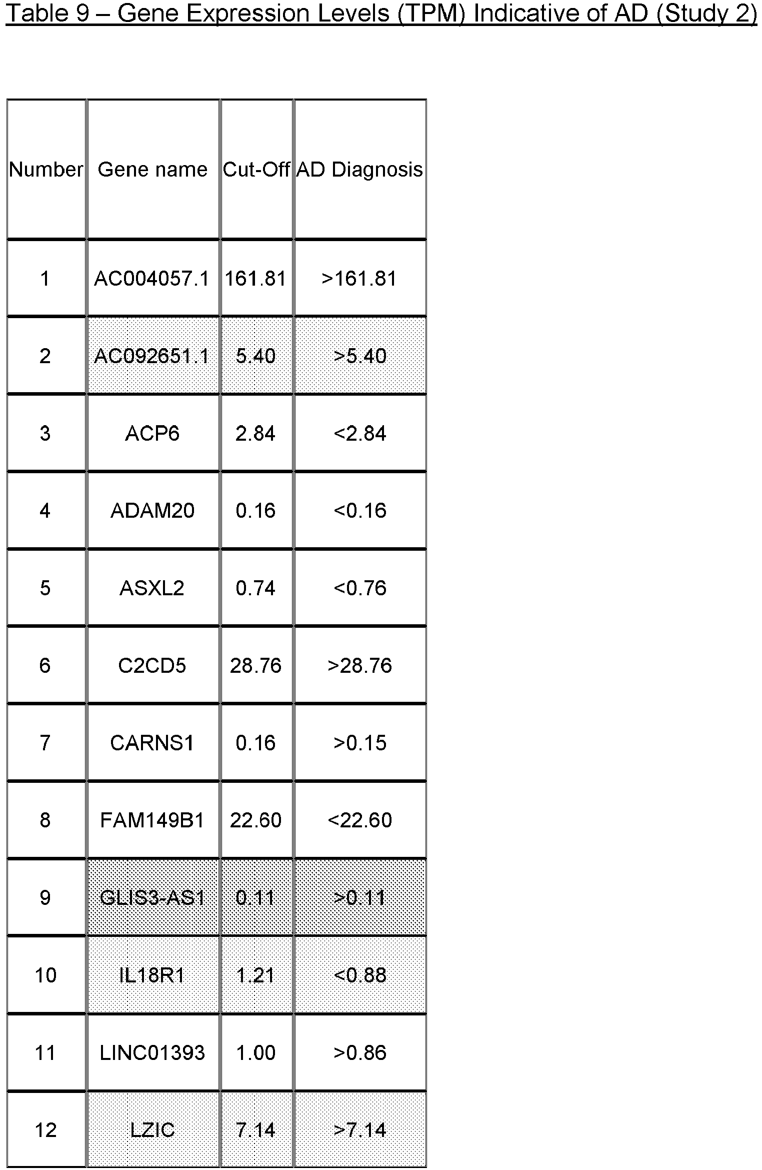

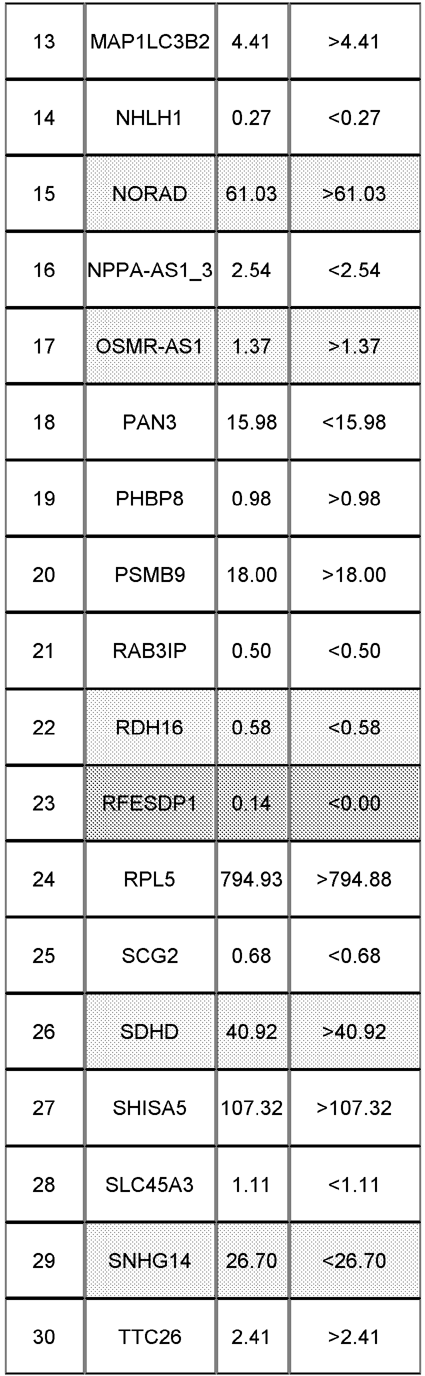

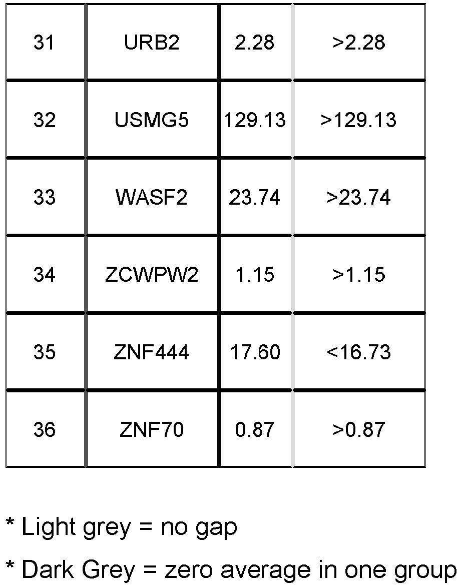

**Table 10 - Gene Expression Levels (TPM) Indicative of AD (Study 2)**

| Number | Gene name | Cut-Off | AD Diagnosis |
|---|---|---|---|
| 1 | AC004057.1 | 161.81 | >161.81 |
| 2 | ACP6 | 2.84 | <2.84 |
| 3 | ADAM20 | 0.16 | <0.16 |
| 4 | ASXL2 | 0.74 | <0.76 |
| 5 | C2CD5 | 28.76 | >28.76 |
| 6 | CARNS1 | 0.16 | >0.15 |
| 7 | FAM149B1 | 22.60 | <22.60 |
| 8 | LINC01393 | 1.00 | >0.86 |
| 9 | MAP1LC3B2 | 4.41 | >4.41 |
| 10 | NHLH1 | 0.27 | <0.27 |
| 11 | NPPA-AS1_3 | 2.54 | <2.54 |
| 12 | PAN3 | 15.98 | <15.98 |
| 13 | PHBP8 | 0.98 | >0.98 |
| 14 | PSMB9 | 18.00 | >18.00 |
| 15 | RAB3IP | 0.50 | <0.50 |
| 16 | RPL5 | 794.93 | >794.88 |
| 17 | SCG2 | 0.68 | <0.68 |
| 18 | SHISA5 | 107.32 | >107.32 |
| 19 | SLC45A3 | 1.11 | <1.11 |
| 20 | TTC26 | 2.41 | >2.41 |
| 21 | URB2 | 2.28 | >2.28 |
| 22 | USMG5 | 129.13 | >129.13 |
| 23 | WASF2 | 23.74 | >23.74 |
| 24 | ZCWPW2 | 1.15 | >1.15 |
| 25 | ZNF444 | 17.60 | <16.73 |
| 26 | ZNF70 | 0.87 | >0.87 |

### References

1. Chen M et al. "Serum Starvation Induced Cell Cycle Synchronization Facilitates Human Somatic Cells Reprogramming", PLoS ONE 7(4) (2012).
2. Baghdadchi N. "The Effects of Serum Starvation on Cell Cycle Synchronization", OSR Journal of Student Research (2013).
3. Hayes O et al. "Cell confluency is as efficient as serum starvation for inducing arrest in the G0/G1 phase of the cell cycle in granulosa and fibroblast cells of cattle", Anim. Reprod. Sci. 87(3-4):181-92 (2005).
4. Spellman PT et al, "Comprehensive identification of cell cycle-regulated genes of the yeast Saccharomyces cerevisiae by microarray hybridization" Mol. Biol. Cell. 9(12):3273-97(1998).

## Claims

1. A method for determining whether a human subject is afflicted with Alzheimer's disease ("AD") or non-Alzheimer's dementia ("non-ADD") when the subject is suspected of being afflicted with AD or non-ADD, comprising the steps of
(a) synchronizing a population of suitable cells derived from the subject; and
(b) in the resulting synchronized cell population, measuring the expression level of a gene known to be differentially expressed between corresponding synchronized cells derived from AD patients and those derived from non-ADD patients, wherein the gene is PHBP8,
whereby (i) the subject is afflicted with AD if the expression level measured in step (b) is consistent with that gene's expression level in corresponding synchronized cells derived from AD patients, and (ii) the subject is afflicted with non-ADD if the expression level measured in step (b) is consistent with that gene's expression level in corresponding synchronized cells derived from non-ADD patients.

2. The method of claim 1, wherein the suitable cells derived from the subject are cultured skin cell fibroblasts.

3. The method of claim 1, wherein the suitable cells derived from the subject are cultured B lymphocytes, preferably wherein the B lymphocytes are immortalized.

4. The method of claim 1, wherein synchronizing the population of suitable cells comprises culturing the cells to over-confluence and then starving the resulting over-confluent cells.

5. The method of claim 1, wherein step (b) comprises measuring the expression levels of a plurality of genes, each gene being known to be differentially expressed between corresponding synchronized cells derived from AD patients and those derived from non-ADD patients preferably, wherein the plurality of genes is selected from the group consisting of at least two genes, at least five genes, at least 20 genes, at least 100 genes, and at least 1,000 genes.

6. The method of claim 5, wherein each gene of the plurality of genes is known to be differentially expressed by at least 50% between corresponding synchronized cells derived from AD patients and those derived from non-ADD patients preferably, wherein each gene of the plurality of genes is known to be differentially expressed by at least 100% between corresponding synchronized cells derived from AD patients and those derived from non-ADD patients.

7. The method of claim 5, wherein the plurality of genes comprises PHBP8 and one or more genes selected from the group consisting of AC004057.1, AC092651.1, ACP6, ADAM20, ASXL2, C2CD5, CARNS1, FAM149B1, GLIS3-AS1, IL18R1, LINC01393, LZIC, MAP1LC3B2, NHLH1, NORAD, NPPA-AS1_3, OSMR-AS1, PAN3, PSMB9, RAB3IP, RDH16, RFESDP1, RPL5, SCG2, SDHD, SHISA5, SLC45A3, SNHG14, TTC26, URB2, USMG5, WASF2, ZCWPW2, ZNF444, and ZNF70.

8. The method of claim 1, wherein measuring the expression level of a gene comprises measuring the number of that gene's RNA transcripts per number of total transcripts.

9. The method of claim 1, comprising the steps of
(a) synchronizing a population of cultured skin cell fibroblasts derived from the subject, wherein the synchronizing comprises culturing the fibroblasts to over-confluence and then starving the resulting over-confluent fibroblasts; and
(b) in the resulting synchronized fibroblast population, measuring the expression level of each of genes AC004057.1, AC092651.1, ACP6, ADAM20, ASXL2, C2CD5, CARNS1, FAM149B1, GLIS3-AS1, IL18R1, LINC01393, LZIC, MAP1LC3B2, NHLH1, NORAD, NPPA-AS1_3, OSMR-AS1, PAN3, PHBP8, PSMB9, RAB3IP, RDH16, RFESDP1, RPL5, SCG2, SDHD, SHISA5, SLC45A3, SNHG14, TTC26, URB2, USMG5, WASF2, ZCWPW2, ZNF444, and ZNF70, wherein measuring the expression level of each gene comprises measuring the number of its RNA transcripts per number of total transcripts,
whereby (i) the subject is afflicted with AD if the expression levels measured in step (b) are consistent with the genes' expression levels in corresponding synchronized cells derived from AD patients, and (ii) the subject is afflicted with non-ADD if the expression levels measured in step (b) are consistent with the genes' expression levels in corresponding synchronized cells derived from non-ADD patients.

10. The method of claim 1, comprising the steps of
(a) synchronizing a population of cultured immortalized B lymphocytes derived from the subject, wherein the synchronizing comprises culturing the lymphocytes to over-confluence and then starving the resulting over-confluent lymphocytes; and
(b) in the resulting synchronized lymphocyte population, measuring the expression level of each of genes AC004057.1, AC092651.1, ACP6, ADAM20, ASXL2, C2CD5, CARNS1, FAM149B1, GLIS3-AS1, IL18R1, LINC01393, LZIC, MAP1LC3B2, NHLH1, NORAD, NPPA-AS1_3, OSMR-AS1, PAN3, PHBP8, PSMB9, RAB3IP, RDH16, RFESDP1, RPL5, SCG2, SDHD, SHISA5, SLC45A3, SNHG14, TTC26, URB2, USMG5, WASF2, ZCWPW2, ZNF444, and ZNF70, wherein measuring the expression level of each gene comprises measuring the number of its RNA transcripts per number of total transcripts,
whereby (i) the subject is afflicted with AD if the expression levels measured in step (b) are consistent with the genes' expression levels in corresponding synchronized cells derived from AD patients, and (ii) the subject is afflicted with non-ADD if the expression levels measured in step (b) are consistent with the genes' expression levels in corresponding synchronized cells derived from non-ADD patients.

## Patentansprüche

1. Verfahren, um festzustellen, ob ein menschliches Subjekt an Alzheimer-Krankheit ("AD") oder an einer Nicht-Alzheimer-Demenz ("Nicht-ADD") leidet, wenn vermutet wird, dass das Subjekt an AD oder Nicht-ADD leidet, umfassend die Schritte:
(a) Synchronisieren einer Population geeigneter Zellen, die von dem Subjekt stammen, und
(b) Messen, in der resultierenden synchronisierten Zellpopulation, des Expressionsniveaus eines Gens, von dem bekannt ist, dass es zwischen entsprechenden synchronisierten Zellen, die von AD-Patienten stammen, und solchen, die von Nicht-ADD-Patienten stammen, unterschiedlich exprimiert wird, wobei das Gen PHBP8 ist,
wobei (i) das Subjekt an AD leidet, wenn das in Schritt (b) gemessene Expressionsniveau mit dem Expressionsniveau dieses Gens in entsprechenden synchronisierten Zellen, die von AD-Patienten stammen, übereinstimmt, und (ii) das Subjekt an Nicht-ADD leidet, wenn das in Schritt (b) gemessene Expressionsniveau mit dem Expressionsniveau dieses Gens in entsprechenden synchronisierten Zellen, die von Nicht-ADD-Patienten stammen, übereinstimmt.

2. Verfahren nach Anspruch 1, wobei die vom Subjekt stammenden geeigneten Zellen kultivierte Hautzellen-Fibroblasten sind.

3. Verfahren nach Anspruch 1, wobei die vom Subjekt stammenden geeigneten Zellen kultivierte B-Lymphozyten sind, vorzugsweise wobei die B-Lymphozyten immortalisiert sind.

4. Verfahren nach Anspruch 1, wobei das Synchronisieren der Population geeigneter Zellen das Kultivieren der Zellen bis zur Überkonfluenz and anschließend das Aushungern der resultierenden überkonfluenten Zellen umfasst.

5. Verfahren nach Anspruch 1, wobei Schritt (b) das Messen der Expressionsniveaus von einer Mehrzahl von Genen umfasst, wobei von jedem Gen bekannt ist, dass es zwischen entsprechenden synchronisierten Zellen, die von AD-Patienten stammen, und solchen, die von Nicht-ADD-Patienten stammen, unterschiedlich exprimiert wird, wobei die Mehrzahl von Genen ausgewählt ist aus der Gruppe bestehend aus wenigstens zwei Genen, wenigstens fünf Genen, wenigstens 20 Genen, wenigstens 100 Genen und wenigstens 1000 Genen.

6. Verfahren nach Anspruch 5, wobei von jedem Gen der Mehrzahl von Genen bekannt ist, dass es zwischen entsprechenden synchronisierten Zellen, die von AD-Patienten stammen, und solchen, die von Nicht-ADD-Patienten stammen, wenigstens 50% unterschiedlich exprimiert wird, vorzugsweise wobei von jedem Gen der Mehrzahl von Genen bekannt ist, dass es zwischen entsprechenden synchronisierten Zellen, die von AD-Patienten stammen, und solchen, die von Nicht-ADD-Patienten stammen, wenigstens 100% unterschiedlich exprimiert wird.

7. Verfahren nach Anspruch 5, wobei die Mehrzahl von Genen PHBP8 und ein oder mehrere Gene, ausgewählt aus der Gruppe bestehend aus AC004057.1, AC092651.1, ACP6, ADAM20, ASXL2, C2CD5, CARNS1, FAM149B1, GLIS3-AS1, IL18R1, LINC01393, LZIC, MAP1LC3B2, NHLH1, NORAD, NPPA-AS1_3, OSMR-AS1, PAN3, PSMB9, RAB3IP, RDH16, RFESDP1, RPL5, SCG2, SDHD, SHISA5, SLC45A3, SNHG14, TTC26, URB2, USMG5, WASF2, ZCWPW2, ZNF444 und ZNF70, umfasst.

8. Verfahren nach Anspruch 1, wobei das Messen des Expressionsniveaus eines Gens das Messen der Anzahl der RNA-Transkripte dieses Gens pro Anzahl der Gesamttranskripte umfasst.

9. Verfahren nach Anspruch 1, umfassend die Schritte:
(a) Synchronisieren einer Population von kultivierten Hautzellen-Fibroblasten, die von dem Subjekt stammen, wobei das Synchronisieren das Kultivieren der Fibroblasten bis zur Überkonfluenz and anschließend das Aushungern der resultierenden überkonfluenten Fibroblasten umfasst, und
(b) Messen, in der resultierenden synchronisierten Fibroblastenpopulation, des Expressionsniveaus eines jeden der Gene AC004057.1, AC092651.1, ACP6, ADAM20, ASXL2, C2CD5, CARNS1, FAM149B1, GLIS3-AS1, IL18R1, LINC01393, LZIC, MAP1LC3B2, NHLH1, NORAD, NPPA-AS1_3, OSMR-AS1, PAN3, PHBP8, PSMB9, RAB3IP, RDH16, RFESDP1, RPL5, SCG2, SDHD, SHISA5, SLC45A3, SNHG14, TTC26, URB2, USMG5, WASF2, ZCWPW2, ZNF444 und ZNF70, wobei das Messen des Expressionsniveaus eines jeden Gens das Messen der Anzahl seiner RNA-Transkripte pro Anzahl der Gesamttranskripte umfasst,
wobei (i) das Subjekt an AD leidet, wenn die in Schritt (b) gemessenen Expressionsniveaus mit den Expressionsniveaus der Gene in entsprechenden synchronisierten Zellen, die von AD-Patienten stammen, übereinstimmen, und (ii) das Subjekt an Nicht-ADD leidet, wenn die in Schritt (b) gemessenen Expressionsniveaus mit den Expressionsniveaus der Gene in entsprechenden synchronisierten Zellen, die von Nicht-ADD-Patienten stammen, übereinstimmen.

10. Verfahren nach Anspruch 1, umfassend die Schritte:
(a) Synchronisieren einer Population von kultivierten immortalisierten B-Lymphozyten, die von dem Subjekt stammen, wobei das Synchronisieren das Kultivieren der Lymphozyten bis zur Überkonfluenz and anschließend das Aushungern der resultierenden überkonfluenten Lymphozyten umfasst, und
(b) Messen, in der resultierenden synchronisierten Lymphozytenpopulation, des Expressionsniveaus eines jeden der Gene AC004057.1, AC092651.1, ACP6, ADAM20, ASXL2, C2CD5, CARNS1, FAM149B1, GLIS3-AS1, IL18R1, LINC01393, LZIC, MAP1LC3B2, NHLH1, NORAD, NPPA-AS1_3, OSMR-AS1, PAN3, PHBP8, PSMB9, RAB3IP, RDH16, RFESDP1, RPL5, SCG2, SDHD, SHISA5, SLC45A3, SNHG14, TTC26, URB2, USMG5, WASF2, ZCWPW2, ZNF444 und ZNF70, wobei das Messen des Expressionsniveaus eines jeden Gens das Messen der Anzahl seiner RNA-Transkripte pro Anzahl der Gesamttranskripte umfasst,
wobei (i) das Subjekt an AD leidet, wenn die in Schritt (b) gemessenen Expressionsniveaus mit den Expressionsniveaus der Gene in entsprechenden synchronisierten Zellen, die von AD-Patienten stammen, übereinstimmen, und (ii) das Subjekt an Nicht-ADD leidet, wenn die in Schritt (b) gemessenen Expressionsniveaus mit den Expressionsniveaus der Gene in entsprechenden synchronisierten Zellen, die von Nicht-ADD-Patienten stammen, übereinstimmen.

## Revendications

1. Méthode permettant de déterminer si un sujet humain est atteint de la maladie d'Alzheimer (« MA ») ou d'une démence de type non Alzheimer (D non A) quand le sujet est soupçonné d'être atteint de la MA ou d'une D non A, comprenant les étapes suivantes :
(a) synchronisation d'une population de cellules appropriées dérivées du sujet ; et
(b) mesure, dans la population de cellules synchronisées obtenue, du taux d'expression d'un gène connu pour être différentiellement exprimé dans des cellules synchronisées correspondantes dérivées de patients MA et celles dérivées de patients D non A, où le gène est PHBP8,
où (i) le sujet est atteint de la MA si le taux d'expression mesuré à l'étape (b) concorde avec le taux d'expression de ce gène dans des cellules synchronisées correspondantes dérivées de patients MA et (ii) le sujet est atteint d'une D non A si le taux d'expression mesuré à l'étape (b) concorde avec le taux d'expression de ce gène dans des cellules synchronisées correspondantes dérivées de patients D non A.

2. Méthode de la revendication 1, où les cellules appropriées dérivées du sujet sont des fibroblastes cutanés en culture.

3. Méthode de la revendication 1, où les cellules appropriées dérivées du sujet sont des lymphocytes B en culture, préférablement où les lymphocytes B sont immortalisés.

4. Méthode de la revendication 1, où la synchronisation de la population de cellules appropriées comprend la culture des cellules à surconfluence puis l'exposition des cellules en surconfluence obtenues à une privation nutritionnelle.

5. Méthode de la revendication 1, où l'étape (b) comprend la mesure des taux d'expression d'une pluralité de gènes, chaque gène étant connu pour être différentiellement exprimé dans des cellules synchronisées correspondantes dérivées de patients MA et celles dérivées de patients D non A, préférablement où la pluralité de gènes est sélectionnée dans le groupe consistant en au moins deux gènes, au moins cinq gènes, au moins 20 gènes, au moins 100 gènes et au moins 1 000 gènes.

6. Méthode de la revendication 5, où chaque gène de la pluralité de gènes est connu pour faire l'objet d'une expression différentielle de 50 % au moins dans des cellules synchronisées correspondantes dérivées de patients MA et celles dérivées de patients D non A, préférablement où chaque gène de la pluralité de gènes est connu pour faire l'objet d'une expression différentielle de 100 % au moins dans des cellules synchronisées correspondantes dérivées de patients MA et celles dérivées de patients D non A.

7. Méthode de la revendication 5, où la pluralité de gènes comprend PHBP8 et un ou plusieurs gènes sélectionnés dans le groupe consistant en les suivants : AC004057.1, AC092651.1, ACP6, ADAM20, ASXL2, C2CD5, CARNS1, FAM149B1, GLIS3-AS1, IL18R1, LINC01393, LZIC, MAP1LC3B2, NHLH1, NORAD, NPPA-AS1_3, OSMR-AS1, PAN3, PSMB9, RAB3IP, RDH16, RFESDP1, RPL5, SCG2, SDHD, SHISA5, SLC45A3, SNHG14, TTC26, URB2, USMG5, WASF2, ZCWPW2, ZNF444 et ZNF70.

8. Méthode de la revendication 1, où la mesure du taux d'expression d'un gène comprend la mesure du nombre de transcripts d'ARN de ce gène rapporté au nombre de transcripts totaux.

9. Méthode de la revendication 1, comprenant les étapes suivantes :
(a) synchronisation d'une population de fibroblastes cutanés en culture dérivés du sujet, où la synchronisation comprend la culture des fibroblastes à surconfluence puis l'exposition des fibroblastes en surconfluence obtenus à une privation nutritionnelle ; et
(b) mesure, dans la population de fibroblastes synchronisés obtenue, du taux d'expression de chacun des gènes suivants : AC004057.1, AC092651.1, ACP6, ADAM20, ASXL2, C2CD5, CARNS1, FAM149B1, GLIS3-AS1, IL18R1, LINC01393, LZIC, MAP1LC3B2, NHLH1, NORAD, NPPA-AS1_3, OSMR-AS1, PAN3, PHBP8, PSMB9, RAB3IP, RDH16, RFESDP1, RPL5, SCG2, SDHD, SHISA5, SLC45A3, SNHG14, TTC26, URB2, USMG5, WASF2, ZCWPW2, ZNF444 et ZNF70, où la mesure du taux d'expression de chaque gène comprend la mesure du nombre de ses transcripts d'ARN rapporté au nombre de transcripts totaux,
où (i) le sujet est atteint de la MA si les taux d'expression mesurés à l'étape (b) concordent avec les taux d'expression des gènes dans des cellules synchronisées correspondantes dérivées de patients MA et (ii) le sujet est atteint d'une D non A si les taux d'expression mesurés à l'étape (b) concordent avec les taux d'expression des gènes dans des cellules synchronisées correspondantes dérivées de patients D non A.

10. Méthode de la revendication 1, comprenant les étapes suivantes :
(a) synchronisation d'une population de lymphocytes B immortalisés en culture dérivés du sujet, où la synchronisation comprend la culture des lymphocytes à surconfluence puis l'exposition des lymphocytes en surconfluence obtenus à une privation nutritionnelle ; et
(b) mesure, dans la population de lymphocytes synchronisés obtenue, du taux d'expression de chacun des gènes suivants : AC004057.1, AC092651.1, ACP6, ADAM20, ASXL2, C2CD5, CARNS1, FAM149B1, GLIS3-AS1, IL18R1, LINC01393, LZIC, MAP1LC3B2, NHLH1, NORAD, NPPA-AS1_3, 0SMR-AS1, PAN3, PHBP8, PSMB9, RAB3IP, RDH16, RFESDP1, RPL5, SCG2, SDHD, SHISA5, SLC45A3, SNHG14, TTC26, URB2, USMG5, WASF2, ZCWPW2, ZNF444 et ZNF70, où la mesure du taux d'expression de chaque gène comprend la mesure du nombre de ses transcripts d'ARN rapporté au nombre de transcripts totaux,
où (i) le sujet est atteint de la MA si les taux d'expression mesurés à l'étape (b) concordent avec les taux d'expression des gènes dans des cellules synchronisées correspondantes dérivées de patients MA et (ii) le sujet est atteint d'une D non A si les taux d'expression mesurés à l'étape (b) concordent avec les taux d'expression des gènes dans des cellules synchronisées correspondantes dérivées de patients D non A.
